# EUROPEAN PATENT SPECIFICATION

(11) **EP 4 291 190 B1**
(45) Date of publication and mention of the grant of the patent: **12.08.2026**
(21) Application number: 22707943.1
(22) Date of filing: 09.02.2022
(51) Int. Cl.: A61K 31/495, A61K 31/519, A61P 35/00

(54) **SEPIAPTERIN IN COMBINATION WITH RADIOTHERAPY OR TEMOZOLOMIDE FOR USE IN TREATING GLIOBLASTOMAS**
SEPIAPTERIN IN KOMBINATION MIT STRAHLENTHERAPIE ODER TEMOZOLOMID ZUR BEHANDLUNG VON GLIOBLASTOMEN
SÉPIAPTÉRINE EN ASSOCIATION AVEC LA RADIOTHÉRAPIE OU LE TÉMOZOLOMIDE POUR LE TRAITEMENT DES GLIOBLASTOMES

(30) Priority: 09.02.2021 US 202163147625 P
(43) Date of publication of application: 20.12.2023
(73) Proprietor: PTC Therapeutics MP, Inc., Warren, NJ 07059 (US); Virginia Commonwealth University, Richmond, VA 23298 (US)
(72) Inventor: RABENDER, Christopher, Chesterfield, VA 23838 (US); CLARK, Gene, Chatman, Richmond, VA 23298 (US); MEZZAROMA, Eleonora, Glen Allen, VA 23059 (US); MIKKELSEN, Ross, B., Richmond, VA 23298 (US); YAKOVLEV, Vasily, Richmond, VA 23298 (US); SMITH, Neil, South Plainfield, NJ 07080 (US)
(74) Representative: Mewburn Ellis LLP
(86) International application number: PCT/US2022/015826
(87) International publication number: WO 2022/173834

(56) References cited:
- WO-A1-2019/175328
- WO-A1-2019/232130
- US-A1- 2016 074 389
- ZHENG XUNZHEN ET AL: "Correction of arginine metabolism with sepiapterin-the precursor of nitric oxide synthase cofactor BH4-induces immunostimulatory-shift of breast cancer", BIOCHEMICAL PHARMACOLOGY, vol. 176, 1 June 2020 (2020-06-01), US, pages 113887, XP055917855, ISSN: 0006-2952, DOI: 10.1016/j.bcp.2020.113887

## Description

### Technical Field

The present invention pertains generally to the field of therapeutic compounds. More specifically, the present invention relates to sepiapterin, or a pharmaceutically acceptable salt thereof, for use in methods of treating glioblastoma in a subject.

### Background of the Invention

Glioblastoma, also known as glioblastoma multiforme (GBM), is an aggressive type of cancer that originates in the brain. Treatment of GBM is difficult, as many drugs cannot cross the blood-brain barrier, and the brain is susceptible to damage from conventional therapies. Radiotherapy is frequently used in treatment of GBM; however, it frequently results in side effects including fatigue, headaches, trouble with memory and speech, seizures, stroke-like symptoms, and poor brain function. In addition, GBM tumors may be resistant to radiotherapy.

Sepiapterin, a metabolic precursor of the enzyme cofactor tetrahydrobiopterin (BH₄), with good oral bioavailability and an excellent safety profile in humans, is currently being tested for the treatment for certain metabolic disorders. Sepiapterin has also been shown to cross the blood brain barrier and have effects on neurotransmitter production. Once sepiapterin is in the brain, it converts to BH₄.

What is needed are methods for treating GBM. What is further needed are agents useful in treating GBM that are capable of crossing the blood-brain barrier. In addition, what is needed are methods for radiosensitizing GBM tumors. In addition, what is further needed are radioprotective agents for treating GBM.

WO 2019/175328 A1 describes a method of increasing T cell activity comprising increasing BH₄ biological activity in said T cell, ex vivo and in vivo, in particular in the treatment of cancer, as well as kits and methods thereof.

Zheng et al., Biochem. Pharm., 2020, Vol. 176, Article No. 113887, describes a study on the supplementation of sepiapterin, and its effect on arginine metabolism, immunogenicity, and the growth of breast tumor cells.

US 2016/0074389 A1 describes methods for treating glioblastoma with temozolomide.

WO 2019/232130 A1 describes compositions including sepiapterin, or a pharmaceutically acceptable salt thereof, and methods for the treatment of BH₄-related disorders.

### Summary of the Invention

Provided herein is sepiapterin, or a pharmaceutically acceptable salt thereof, for use in methods of treating glioblastoma in a subject, comprising administering to the subject an effective amount of the sepiapterin or a pharmaceutically acceptable salt thereof, wherein the method further comprises: treating the subject with therapeutic radiation; and/or treating the subject with temozolomide (TMZ). In some embodiments, the effective amount of sepiapterin, or pharmaceutically acceptable salt thereof, is about 10 mg/kg to about 60 mg/kg per dose. In some embodiments, the effective amount of sepiapterin, or pharmaceutically acceptable salt thereof, is about 20 mg/kg to about 60 mg/kg per dose. In some embodiments, the effective amount of sepiapterin, or pharmaceutically acceptable salt thereof, is about 20 mg/kg per dose. In some embodiments, the effective amount of sepiapterin, or pharmaceutically acceptable salt thereof, is about 40 mg/kg per dose. In some embodiments, the effective amount of sepiapterin, or pharmaceutically acceptable salt thereof, is about 60 mg/kg per dose. In some embodiments, including any of the foregoing embodiments, the effective amount of sepiapterin, or pharmaceutically acceptable salt thereof, is administered once daily. In some embodiments, including any of the foregoing embodiments, the effective amount of sepiapterin, or pharmaceutically acceptable salt thereof, is administered twice daily. In some embodiments, including any of the foregoing embodiments, the effective amount of sepiapterin, or pharmaceutically acceptable salt thereof, is administered in two equal doses. In some embodiments, including any of the foregoing embodiments, the effective amount of sepiapterin, or a pharmaceutically acceptable salt thereof, is administered with food. In some embodiments, including any of the foregoing embodiments, administration to the subject occurs less than 30 minutes prior to consuming food or after consuming food. In some embodiments, including any of the foregoing embodiments, administration to the subject is substantially at the same time as food. In some embodiments, including any of the foregoing embodiments, the food is high protein and/or high fat food. In some embodiments, including any of the foregoing embodiments, the food is a low fat food. In some embodiments, including any of the foregoing embodiments, the food is high calorie food. In some embodiments, including any of the foregoing embodiments, the effective amount of sepiapterin, or a pharmaceutically acceptable salt thereof, is administered without food. In some embodiments, including any of the foregoing embodiments, administration to the subject occurs more than 30 minutes prior to consuming food or more than 2 hours after consuming food. In some embodiments, including any of the foregoing embodiments, administration to the subject occurs more than 30 minutes prior to consuming food or more than 3 hours after consuming food. In some embodiments, including any of the foregoing embodiments, the sepiapterin or a pharmaceutically acceptable salt thereof, is formulated as an oral powder for suspension. In some embodiments, including any of the foregoing embodiments, the sepiapterin or a pharmaceutically acceptable salt thereof, is administered as a suspension in a flavored suspending vehicle. In some embodiments, including any of the foregoing embodiments, the sepiapterin or a pharmaceutically acceptable salt thereof, is administered as a suspension in water or juice (e.g., apple, orange, grape, etc.). In some embodiments, including any of the foregoing embodiments, the sepiapterin or a pharmaceutically acceptable salt thereof, is formulated as an oral tablet, capsule, or caplet. In some embodiments, including any of the foregoing embodiments, the method further comprises treating the subject with therapeutic radiation. In some embodiments, including any of the foregoing embodiments, the sepiapterin or a pharmaceutically acceptable salt thereof is administered during the duration of the therapeutic radiation. In some embodiments, including any of the foregoing embodiments, the sepiapterin or a pharmaceutically acceptable salt thereof is administered prior to the therapeutic radiation. In some embodiments, including any of the foregoing embodiments, the sepiapterin or a pharmaceutically acceptable salt thereof is administered subsequent to the therapeutic radiation. In some embodiments, including any of the foregoing embodiments, the sepiapterin or a pharmaceutically acceptable salt thereof is administered for at least 5 days. In some embodiments, including any of the foregoing embodiments, the sepiapterin or a pharmaceutically acceptable salt thereof is administered for at least 6 days. In some embodiments, including any of the foregoing embodiments, the sepiapterin or a pharmaceutically acceptable salt thereof is administered for at least 10 days. In some embodiments, including any of the foregoing embodiments, the sepiapterin or a pharmaceutically acceptable salt thereof is administered for at least 14 days. In some embodiments, including any of the foregoing embodiments, the sepiapterin or a pharmaceutically acceptable salt thereof is administered for at least 15 days. In some embodiments, including any of the foregoing embodiments, the sepiapterin or a pharmaceutically acceptable salt thereof is administered for at least 30 days. In some embodiments, including any of the foregoing embodiments, the sepiapterin or pharmaceutically acceptable salt thereof is administered at least 6 days, or at least 10 days, or at least 14 days, or at least 30 days prior to the therapeutic radiation. In some embodiments, including any of the foregoing embodiments, the sepiapterin or pharmaceutically acceptable salt thereof is administered at least 6 days, or at least 10 days, or at least 14 days, or at least 30 days subsequent to the therapeutic radiation. In some embodiments, including any of the foregoing embodiments, the sepiapterin or a pharmaceutically acceptable salt thereof is administered continuously concurrent to the therapeutic radiation. In some embodiments, including any of the foregoing embodiments, the sepiapterin or a pharmaceutically acceptable salt thereof is administered daily for a 28-day cycle of treatment. In some embodiments, including any of the foregoing embodiments, the sepiapterin or a pharmaceutically acceptable salt thereof is administered daily for consecutive 28-day cycles of treatment. In some embodiments, including any of the foregoing embodiments, the sepiapterin or a pharmaceutically acceptable salt thereof is administered daily for at least six 28-day cycles of treatment. In some embodiments, including any of the foregoing embodiments, the sepiapterin or a pharmaceutically acceptable salt thereof is administered daily for consecutive 28-day cycles of treatment until Progressive Disease (PD) occurs. In some embodiments, including any of the foregoing embodiments, the sepiapterin or a pharmaceutically acceptable salt thereof is administered daily for consecutive 28-day cycles of treatment until death. In some embodiments, including any of the foregoing embodiments, the method further comprises treating the subject with temozolomide (TMZ). In some embodiments, including any of the foregoing embodiments, the subject is a human.

In some embodiments of any of the methods described herein, administering sepiapterin, or a pharmaceutically acceptable salt thereof, produces a BH₄ concentration of at least 50 ng/mL (e.g., at least 60 ng/mL, at least 100 ng/mL, at least 200 ng/mL, at least 400 ng/mL, at least 600 ng/mL, at least 1000 ng/mL, or at least 2000 ng/mL or from 50 ng/mL to 100 ng/mL from 60 ng/mL to 400 ng/mL, from 200 ng/mL to 600 ng/mL, from 400 ng/mL to 1000 ng/mL, or from 600 ng/mL to 1500 ng/mL) in the plasma of the subject within 10 hours of administration.

In some embodiments of any of the methods described herein, the effective amount is an amount (e.g., 2.5 mg/kg to 100 mg/kg per dose) sufficient to produce a BH₄ concentration of at least 50 ng/mL (e.g., at least 60 ng/mL, at least 100 ng/mL, at least 200 ng/mL, at least 400 ng/mL, at least 600 ng/mL, at least 1000 ng/mL, or at least 2000 ng/mL, or from 50 ng/mL to 100 ng/mL from 60 ng/mL to 400 ng/mL, from 200 ng/mL to 600 ng/mL, from 400 ng/mL to 1000 ng/mL, or from 600 ng/mL to 1500 ng/mL) in the plasma of the subject within 10 hours of administration of the sepiapterin or a pharmaceutically acceptable salt thereof.

In some embodiments of any of the methods described herein, the effective amount of sepiapterin, or a pharmaceutically acceptable salt thereof, is about 2.5 mg/kg to 100 mg/kg per dose (e.g., about 20 mg/kg to about 60 mg/kg, or about 20 mg/kg, about 30 mg/kg, about 40 mg/kg, about 50 mg/kg, about 60 mg/kg).

In some embodiments of any of the methods described herein, the effective amount of sepiapterin, or a pharmaceutically acceptable salt thereof, is administered with food. In some embodiments of any of the methods described herein, the effective amount is an amount (e.g., 1 mg/kg to 100 mg/kg per dose, or 2.5 mg/kg to 100 mg/kg per dose) sufficient to produce a BH₄ concentration of at least 50 ng/mL (e.g., at least 60 ng/mL, at least 100 ng/mL, at least 200 ng/mL, at least 400 ng/mL, at least 600 ng/mL, at least 1000 ng/mL, or at least 2000 ng/mL, or from 50 ng/mL to 100 ng/mL from 60 ng/mL to 400 ng/mL, from 200 ng/mL to 600 ng/mL, from 400 ng/mL to 1000 ng/mL, or from 600 ng/mL to 1500 ng/mL) in the plasma of the subject within 10 hours of administration with food. In some embodiments, the effective amount includes a dose that is at least 5% (at least 10%, at least 20%, at least 50%, at least 70%, at least 90%, at least 100%, at least 110%, at least 120%, at least 130%, at least 140%, or at least 150%) lower than the dose sufficient to produce a maximum BH4 plasma concentration (Cmax) of at least 50 ng/mL (e.g., at least 60 ng/mL, at least 100 ng/mL, at least 200 ng/mL, at least 400 ng/mL, at least 600 ng/mL, at least 1000 ng/mL, or at least 2000 ng/mL or from 50 ng/mL to 100 ng/mL from 60 ng/mL to 400 ng/mL, from 200 ng/mL to 600 ng/mL, from 400 ng/mL to 1000 ng/mL, or from 600 ng/mL to 1500 ng/mL) in the plasma of the subject within 10 hours of administration of sepiapterin, or a pharmaceutically acceptable salt thereof, without food.

In some embodiments of any of the methods described herein, administration to the subject occurs less than 30 minutes prior to consuming food, or after consuming food, e.g., immediately prior to the consumption of food or up to 1 hour after consumption. In some embodiments, the administration to the subject is substantially at the same time as food. In some embodiments of any of the methods described herein, the food is a high protein food. In some embodiments of any of the methods described herein, the food is a high fat food (e.g., at least 25, 30, 40, or 50% of the calories are from fat). In some embodiments of any of the methods described herein, the food is a high protein and high fat food. In some embodiments, the food is high calorie food (e.g., the food includes at least 100 calories, e.g., at least 200 calories, at least 300 calories, at least 400 calories, at least 500 calories, e.g., 500-1500 or 800-1000 calories). In some embodiments of any of the methods described herein, the food is a meal, e.g., breakfast, lunch, or dinner. In some embodiments of any of the methods described herein, the food is a low fat food (e.g. no more than 25% of the calories are from fat).

In some embodiments of any of the methods described herein, the administration with food (e.g., occurring less than 30 minutes prior to consuming food, or after consuming food, e.g., immediately prior to the consumption of food up to 1 hour after consumption) results in an increase (e.g., at least 5%, at least 10%, at least 20%, at least 30%, at least 40%, at least 50%, at least 60%, at least 70%, at least 80%, at least 90%, at least 100%, at least 110%, at least 120%, at least 130%, at least 140%, or at least 150%) in the Cmax of BH₄ compared to administration without food (e.g., occurring more than 2 hours after consuming food until 30 minutes prior to consuming further food).

In some embodiments of any of the methods described herein, the administration with food (e.g., occurring less than 30 minutes prior to consuming food or after consuming food, e.g., immediately prior to the consumption of food up to 1 hour after consumption) results in an increase (e.g., at least 5%, at least 10%, at least 20%, at least 30%, at least 40%, at least 50%, at least 60%, at least 70%, at least 80%, at least 90%, at least 100%, at least 110%, at least 120%, at least 130%, at least 140%, or at least 150%) in the extent of production and resulting plasma exposure (AUC₀₋ₗₐₛₜ) of BH₄ compared to administration without food (e.g., occurring less than 30 minutes prior to consuming food or after consuming food, e.g., immediately prior to the consumption of food up to 1 hour after consumption).

In some embodiments of any of the methods described herein, the sepiapterin, or a pharmaceutically acceptable salt thereof, is provided in a separate composition from the consumed food (e.g., the sepiapterin, or a pharmaceutically acceptable salt thereof, is not incorporated into a food product). In some embodiments of any of the methods described herein, the consumption of food occurs prior to the administration of sepiapterin or a pharmaceutically acceptable salt thereof (e.g., the consumption of food occurs between 1 hour up to immediately prior to the administration of sepiapterin, or a pharmaceutically acceptable salt thereof). In some embodiments of any of the methods described herein, the consumption of food occurs after the administration of sepiapterin or a pharmaceutically acceptable salt thereof (e.g., the consumption of food occurs between immediately after administration up to 30 minutes after administration).

In some embodiments of any of the foregoing methods, the effective amount is an amount (e.g., 2.5 mg/kg to 100 mg/kg per dose) sufficient to produce a sepiapterin plasma concentration of at least 0.5 ng/mL (e.g., at least 1 ng/mL, at least 1.5 ng/mL, at least 2.5 ng/mL, or at least 3.5 ng/mL) in the plasma of the subject within 1 hour of administration without food, e.g., the effective amount includes a dose that is at least 10% (e.g., at least 20%, at least 40%, at least 60%, at least 80%, at least 100%, or at least 120%) lower than the dose sufficient to produce a maximum plasma concentration (Cmax) of at least 0.5 ng/mL (e.g., at least 1 ng/mL, at least 1.5 ng/mL, at least 2.5 ng/mL, or at least 3.5 ng/mL) in the plasma of the subject within 1 hour of administration of sepiapterin with food. In some embodiments, the administration (e.g., occurring more than about 30 minutes prior to or at least 2 hours after consuming food) results in an increase (e.g., at least 10% (at least 20%, at least 40%, at least 60%, at least 80%, at least 100%, or at least 120%) in the maximum plasma, CSF, and/or brain concentration (Cmax) of sepiapterin compared to administration with food (e.g., occurring less than 30 minutes prior to 2 hours after consuming food). In some embodiments, the administration (e.g., occurring more than 30 minutes prior or at least 2 hours after consuming food) results in an increase (e.g., at least 10% (at least 20%, at least 40%, at least 60%, at least 80%, at least 100%, or at least 120%) in the extent of absorption (AUC₀₋ₗₐₛₜ) of sepiapterin compared to administration with food (e.g., the administration to the subject occurs less than 30 minutes prior to less than 2 hours after consuming food).

In some embodiments of any of the methods described herein, the effective amount of sepiapterin, or a pharmaceutically acceptable salt thereof, is administered in two equal doses (e.g., two doses at different times of day). In some embodiments of any of the methods described herein, the effective amount of sepiapterin, or a pharmaceutically acceptable salt thereof, is administered once per day. In some embodiments of any of the methods described herein, the effective amount of sepiapterin, or a pharmaceutically acceptable salt thereof, is administered in two 60 mg/kg doses (e.g., one 60 mg/kg dose in the morning and one 60 mg/kg dose in the evening). In some embodiments of any of the methods described herein, the effective amount of sepiapterin, or a pharmaceutically acceptable salt thereof, is administered in two 40 mg/kg doses (e.g., one 40 mg/kg dose in the morning and one 40 mg/kg dose in the evening). In some embodiments of any of the methods described herein, the effective amount of sepiapterin, or a pharmaceutically acceptable salt thereof, is administered in two 30 mg/kg doses (e.g., one 30 mg/kg dose in the morning and one 30 mg/kg dose in the evening). In some embodiments of any of the methods described herein, the effective amount of sepiapterin, or a pharmaceutically acceptable salt thereof, is administered in two 20 mg/kg doses (e.g., one 20 mg/kg dose in the morning and one 20 mg/kg dose in the evening). In some embodiments of any of the methods described herein, the effective amount of sepiapterin, or a pharmaceutically acceptable salt thereof, is administered in two 10 mg/kg doses (e.g., one 10 mg/kg dose in the morning and one 10 mg/kg dose in the evening).

In embodiments of any of the methods described herein, the method includes administering to the subject an effective amount of sepiapterin, or a pharmaceutically acceptable salt thereof, with food once per day. In embodiments of any of the methods described herein, the method includes administering to the subject an effective amount of sepiapterin, or a pharmaceutically acceptable salt thereof, with food more than once per day, e.g., twice per day. In embodiments of any of the methods described herein, the method includes administering to the subject an effective amount of sepiapterin, or a pharmaceutically acceptable salt thereof, without food once per day. In embodiments of any of the methods described herein, the method includes administering to the subject an effective amount of sepiapterin, or a pharmaceutically acceptable salt thereof, without food more than once per day, e.g., twice per day.

In some embodiments of any of the methods described herein, the subject is a child (e.g. the subject is less than 18 years old, less than 17 years old, less than 16 years old, less than 15 years old, less than 14 years old, less than 13 years old, less than 12 years old, less than 11 years old, less than 10 years old, less than 9 years old, less than 8 years old, less than 7 years old, less than 6 years old, less than 5 years old, less than 4 years old, less than 3 years old, less than 2 years old, less than 1 year old). In some embodiments of any of the methods described herein, the subject is an adult (e.g., the subject is greater than 18 years old). In some embodiments, the subject is greater than 20 years old, greater than 30 years old, greater than 40 years old, greater than 50 years old, greater than 60 years old, greater than 7years old, greater than 80 years old, greater than 90 years old.

In some embodiments of any of the methods described herein, the sepiapterin or a pharmaceutically acceptable salt thereof, is formulated as an oral powder for suspension. In some embodiments of any of the methods described herein, the sepiapterin or a pharmaceutically acceptable salt thereof, is administered as a suspension in a flavored suspending vehicle (e.g., MEDISCA^{®}Oral Mix). In some embodiments of any of the methods described herein, the sepiapterin or a pharmaceutically acceptable salt thereof, is administered as a suspension in water or juice (e.g., apple juice). In some embodiments of any of the methods described herein, the sepiapterin or a pharmaceutically acceptable salt thereof, is administered as a suspension a food such as apple sauce or pudding. In some embodiments of any of the methods described herein, the sepiapterin or pharmaceutically acceptable salt thereof is formulated as a tablet, capsule, or caplet.

### Definitions

In this application, unless otherwise clear from context, (i) the term "a" may be understood to mean "at least one"; (ii) the term "or" may be understood to mean "and/or"; (iii) the terms "comprising" and "including" may be understood to encompass itemized components or steps whether presented by themselves or together with one or more additional components or steps; and (iv) the term "approximately" may be understood to permit standard variation as would be understood by those of ordinary skill in the art; and (v) where ranges are provided, endpoints are included.

It is to be understood that the description of compounds, compositions, formulations, and methods of treatment described herein include "comprising", "consisting of", and "consisting essentially of" embodiments. In some embodiments, for all compositions described herein, and all methods using a composition described herein, the compositions can either comprise the listed components or steps, or can "consist essentially of" the listed components or steps. When a composition is described as "consisting essentially of" the listed components, the composition contains the components listed, and may contain other components which do not substantially affect the condition being treated, but do not contain any other components which substantially affect the condition being treated other than those components expressly listed; or, if the composition does contain extra components other than those listed which substantially affect the condition being treated, the composition does not contain a sufficient concentration or amount of the extra components to substantially affect the condition being treated. When a method is described as "consisting essentially of" the listed steps, the method contains the steps listed, and may contain other steps that do not substantially affect the condition being treated, but the method does not contain any other steps which substantially affect the condition being treated other than those steps expressly listed. As a non-limiting specific example, when a composition is described as 'consisting essentially of' a component, the composition may additionally contain any amount of pharmaceutically acceptable carriers, vehicles, or diluents and other such components which do not substantially affect the condition being treated.

Unless otherwise clear from context, all references to sepiapterin contained herein refer to sepiapterin or a pharmaceutically acceptable salt of sepiapterin.

As used herein, the term "about" represents a value that is in the range of ±10% of the value that follows the term "about." Reference to "about" a value or parameter herein includes (and describes) variations that are directed to that value or parameter per se. For example, description referring to "about X" includes description of "X".

As used herein, the term "administration" refers to the administration of a composition (e.g., a compound or a preparation that includes a compound as described herein) to a subject or system. Administration to an animal subject (e.g., to a human) may be by any appropriate route. For example, in some embodiments, administration may be bronchial (including by bronchial instillation), buccal, enteral, interdermal, intra-arterial, intradermal, intragastric, intramedullary, intramuscular, intranasal, intraperitoneal, intrathecal, intravenous, intraventricular, mucosal, nasal, oral, rectal, subcutaneous, sublingual, topical, tracheal (including by intratracheal instillation), transdermal, vaginal, and vitreal.

An "effective amount" of a compound may vary according to factors such as the disease state, age, sex, and weight of the individual, and the ability of the compound to elicit the desired response. A therapeutically effective amount encompasses an amount in which any toxic or detrimental effects of the compound are outweighed by the therapeutically beneficial effects. An effective amount also encompasses an amount sufficient to confer benefit, e.g., clinical benefit.

The term "food," as used herein, refers to solid food with sufficient bulk and fat content that it is not rapidly dissolved and absorbed in the stomach. For example, a meal, such as breakfast, lunch, or dinner. The term "with food," as used herein refers to administration of a composition between about 30 minutes prior to to about two hours after eating, e.g., a meal. The terms "without food," "fasted," or "an empty stomach" refer to the condition of not having consumed solid food for at least about 2 hours after (e.g. at least 3 hours after) until about 30 minutes prior to consuming further solid food.

"Low-fat food" indicates a meal having no more than 25% of calories from fat. In some embodiments, the low-fat meal contains about 11-14 g of fat. In some embodiments, the low-fat food contains about 400-500 total calories.

By "natural protein" is meant protein from a natural source (e.g., animal, plant, or fungus).

The term "pharmaceutical composition," as used herein, represents a composition containing a compound described herein formulated with a pharmaceutically acceptable excipient. Pharmaceutical compositions can be formulated, for example, for oral administration in unit dosage form (e.g., a tablet, capsule, caplet, gel cap, suspension, solution, or syrup); for topical administration (e.g., as a cream, gel, lotion, or ointment); for intravenous administration (e.g., as a sterile solution free of particulate emboli and in a solvent system suitable for intravenous use); or in any other pharmaceutically acceptable formulation.

As used herein, the term "pharmaceutically acceptable salt" means any salt that within the scope of sound medical judgment is suitable for use in contact with the tissues of humans and animals without undue toxicity, irritation, allergic response and are commensurate with a reasonable benefit/risk ratio. Pharmaceutically acceptable salts are well known in the art. For example, pharmaceutically acceptable salts are described in: Berge et al., J. Pharmaceutical Sciences 66:1-19, 1977 and in Pharmaceutical Salts: Properties, Selection, and Use, (Eds. P.H. Stahl and C.G. Wermuth), Wiley-VCH, 2008. The salts can be prepared in situ during the final isolation and purification of the compounds described herein or separately by reacting a free base group with a suitable organic acid.

Frequently, the compounds are prepared or used as pharmaceutically acceptable salts prepared as addition products of pharmaceutically acceptable acids. Suitable pharmaceutically acceptable acids and methods for preparation of the appropriate salts are well-known in the art. Salts may be prepared from pharmaceutically acceptable non-toxic acids including inorganic and organic acids.

Representative acid addition salts include acetate, adipate, alginate, ascorbate, aspartate, benzenesulfonate, benzoate, besylate, bisulfate, borate, butyrate, camphorate, camphorsulfonate, citrate, cyclopentanepropionate, digluconate, dodecylsulfate, ethanesulfonate, fumarate, gentisate, glucoheptonate, glycerophosphate, glycolate, hemisulfate, heptonate, hexanoate, hydrobromide, hydrochloride, hydroiodide, 2-hydroxyethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, undecanoate, and valerate salts.

As used herein, the term "substantially free" refers to the qualitative condition of exhibiting total or near-total extent or degree of the absence of a compound or type of compound of interest. One of ordinary skill in the biological arts will understand that biological and chemical phenomena rarely, if ever, can be determined to be zero without doubt, e.g., due to inherent error in any measurement. The term "substantially free" is therefore used herein to capture the potential lack of completeness inherent in many biological and chemical measurements.

As used herein, the term "subject" or "participant" or "patient" refers to any organism to which a compound or composition in accordance with the invention may be administered, e.g., for experimental, diagnostic, prophylactic, and/or therapeutic purposes. Typical subjects include any animal (e.g., mammals such as mice, rats, rabbits, non-human primates, and humans). A subject may seek or be in need of treatment, require treatment, be receiving treatment, be receiving treatment in the future, or be a human or animal who is under care by a trained professional for a particular disease or condition.

As used herein, the terms "treat," "treated," or "treating" mean both therapeutic treatment and prophylactic or preventative measures wherein the object is to prevent or slow down (lessen) an undesired physiological condition, disorder, or disease, or obtain beneficial or desired clinical results. Beneficial or desired clinical results include, but are not limited to, alleviation of symptoms; diminishment of the extent of a condition, disorder, or disease; stabilized (i.e., not worsening) state of condition, disorder, or disease; delay in onset or slowing of condition, disorder, or disease progression; amelioration of the condition, disorder, or disease state or remission (whether partial or total), whether detectable or undetectable; an amelioration of at least one measurable physical parameter, not necessarily discernible by the subject; or enhancement or improvement of condition, disorder, or disease. Treatment includes eliciting a clinically significant response without excessive levels of side effects. Treatment also includes prolonging survival as compared to expected survival if not receiving treatment.

"Progressive Disease (PD)" indicates one or more of the following: (1) appearance of one or more new lesions, (2) at least a 20% increase in the sum of diameters of target lesions, taking as reference the smallest sum on study (this includes the baseline sum if that is the smallest on study); in addition to the relative increase of 20%, the sum must also demonstrate an absolute increase of at least 5 mm. Eisenhauer EA, Therasse P, Bogaerts J et al. New response evaluation criteria in solid tumours: revised RECIST guideline (versions 1.1). Eur J Cancer. 2009; 45:228-247.

Unless otherwise defined, all technical and scientific terms used herein have the same meaning as commonly understood by one of ordinary skill in the art to which this invention belongs. Methods and materials are described herein for use in the present disclosure; other, suitable methods and materials known in the art can also be used. The materials, methods, and examples are illustrative only and not intended to be limiting. In case of conflict, the present specification, including definitions, will control.

The details of one or more embodiments of the invention are set forth in the description below. Other features, objects, and advantages of the invention will be apparent from the description and from the claims.

### Brief Description of the Drawings

**FIG. 1** is a graph showing survival of nude (U87) mice having glioblastoma tumors, as treated with sepiapterin in combination with irradiation or irradiation alone compared with control.
**FIG. 2** is a graph showing survival of syngenic (GL261) mice, as treated with sepiapterin (SP) alone, sepiapterin in combination with radiation (IR) or with radiation compared to control.
**FIG. 3** is a graph showing survival of mice having glioblastoma tumors, as treated with temozolomide (TMZ) alone or with sepiapterin compared to control.
**FIG. 4** is a graph showing Novel Object Recognition (NOR) Discrimination Index of mice treated with either radiation or radiation and sepiapterin, versus control (naïve).
**FIG. 5** is a schematic of a Phase 2 study of sepiapterin in combination with temozolomide and therapeutic radiation in newly diagnosed or recurrent glioblastoma.

### Detailed Description

Any references herein to methods of treatment by therapy or surgery or in vivo diagnosis are to be interpreted as references to compounds, pharmaceutical compositions and medicaments described herein for use in those methods.

The present inventors have discovered that sepiapterin is effective in treating patients with glioblastoma. In some embodiments, the sepiapterin is administered with food. In some embodiments, the sepiapterin is administered without food. Without wishing to be bound by theory, sepiapterin may not only sensitize glioblastoma to therapeutic radiation and/or chemotherapeutic agents; it may also protect the brain against side effects from therapeutic radiation and/or chemotherapeutic agents. In some embodiments, sepiapterin prevents, delays, and/or mitigates decline in cognitive function due to therapeutic radiation and/or chemotherapeutic agents. Accordingly, the present invention features sepiapterin, or a pharmaceutically acceptable salt thereof, for use in a method for the treatment of glioblastoma in a subject, the method comprising administering to the subject an effective amount of sepiapterin, or a pharmaceutically acceptable salt thereof, wherein the method further includes treatment with a chemotherapeutic agent and/or therapeutic radiation.

### Sepiapterin

Sepiapterin ((S)-2-amino-6-(2-hydroxypropanoyl)-7,8-dihydropteridin-4(3H)-one) passes into the cell and is converted to 7,8-dihydrobiopterin by sepiapterin reductase. 7,8-dihydrobiopterin is then converted to BH4 via reduction by dihydrofolate reductase.

Sepiapterin, or a pharmaceutically acceptable salt thereof, can be formulated in a pharmaceutical composition. In some embodiments, a pharmaceutical composition of the invention includes 20-30% sepiapterin, or a salt thereof, by total weight, e.g., about 20%, 22%, 25%, 27%, or 30%. In some embodiments, the pharmaceutical compositions include greater than 20% sepiapterin by total weight, e.g., greater than 25%, greater than 30%, greater than 40%, greater than 50%, greater than 60%, greater than 70%, greater than 80%, or greater than 90%. In some embodiments, the pharmaceutical composition includes less than 20% sepiapterin by total weight, e.g., less than 20%, less than 15%, less than 10%, or less than 5%.

In some embodiments, the invention features a pharmaceutical composition including sepiapterin, or a pharmaceutically acceptable salt thereof, and less than 10% by total weight of an antioxidant, e.g., about 9%, 7%, 5%, 3%, 1%, 0.5%, 0.25%, 0.1%, or no antioxidant. The antioxidant may be ascorbic acid. In some embodiments, the ratio of sepiapterin, or a pharmaceutically acceptable salt thereof, to the antioxidant is 1:1, or greater than 1:1, e.g., 2:1, 5:1, 7:1, or 10:1 by weight. The pharmaceutical composition may include 20-30% sepiapterin, or a pharmaceutically acceptable salt thereof, by total weight, e.g., about 20%, 22%, 25%, 27%, or 30%. The pharmaceutical composition can further include a dispersant, e.g., croscarmellose sodium. The pharmaceutical composition may include 0.1-1.5% dispersant by total weight, e.g., 0.1%, 0.5%, 1%, or 1.5%. In some embodiments, the pharmaceutical composition includes at least one anti-caking agent, e.g., colloidal silicon dioxide or microcrystalline cellulose. The pharmaceutical composition may include 65-75% anti-caking agent by total weight, e.g., about 65%, 67%, 70%, 73%, or 75%. In some embodiments, the pharmaceutical composition includes both colloidal silicon dioxide and microcrystalline cellulose. In some embodiments, the pharmaceutical composition includes 60-65% microcrystalline cellulose by total weight and 5-7% colloidal silicon dioxide by total weight. In some embodiments, the crystalline form of sepiapterin is formulated as particles less than 140 µm in size, e.g., about 120 µm, 110 µm, 100 µm, 90 µm, 80 µm, 70 µm, 60 µm, 50 µm, 40 µm, 30 µm, 20 µm, 10 µm, or 5 µm. In some embodiments, the pharmaceutical composition includes less than 1% of an impurity by total weight, such as lactoylpterin, e.g., the composition includes less than 0.9%, less than 0.8%, less than 0.7%, less than 0.6%, less than 0.5%, less than 0.4%, less than 0.3%, or less than 0.2%.

In some embodiments, the sepiapterin is a salt of sepiapterin, e.g., with sulfuric acid, p-toluene sulfonic acid, methane sulfonic acid, benzene sulfonic acid, malonic acid, tartaric acid (e.g., L-tartaric acid), phosphoric acid, gentisic acid, fumaric acid, glycolic acid, acetic acid, or nicotinic acid.

In some embodiments, the sepiapterin, or pharmaceutically acceptable salt thereof, is in crystalline form. The crystalline sepiapterin free base or a crystalline form of a salt of sepiapterin can occur as an anhydrate (e.g., without having any bound water or solvent or hydration or solvation) or as a hydrate, a partial hydrate (e.g., hemihydrate, sesquihydrate, and the like), as a dihydrate, a trihydrate, or the like, wherein the crystalline form binds a water of hydration or a solvent molecule associated with the crystalline form of sepiapterin or salt thereof. In an embodiment, crystalline sepiapterin occurs as a monohydrate or as a hemihydrate.

Exemplary salts, co-crystals, and crystalline forms of sepiapterin are described in WO 2018/102314, WO 2018/102315, WO 2019/232120, and WO 2019/046849.

In some embodiments, the crystalline form of sepiapterin free base is crystalline Form F of sepiapterin free base and is characterized by an X-ray powder diffraction pattern obtained by irradiation with Cu Kα X-rays having peaks expressed as 2θ at 9.7°±0.5, e.g., 9.7 °±0.2, 10.2°±0.5, e.g., 10.2°±0.2, and 11.3°±0.5, e.g., 11.3°±0.2. In other embodiments, the crystalline form of sepiapterin is characterized by an X-ray powder diffraction pattern obtained by irradiation with Cu Kα X-rays having peaks expressed as 2θ at 9.7°±0.5, e.g., 9.7°±0.2, 10.2°±0.5, e.g., 10.2°±0.2, 11.3°±0.5, e.g., 11.3°±0.2, 14.0°±0.5, e.g., 14.0°±0.2, 14.6°±0.5, e.g., 14.6°±0.2, 19.9°±0.5, e.g., 19.9°±0.2, 22.2°±0.5, e.g., 22.2°±0.2, 25.3°±0.5, e.g., 25.3°±0.2, and 32.4°±0.5, e.g., 32.4°±0.2. In an essentially pure form of this crystalline form, peaks can be observed at angles of refraction 2θ as set forth in Table 1. Alternatively or in addition, this crystalline form is characterized by a DSC curve showing two endotherms at 71.6° C and 233.4° C.

**Table 1**

| **Position [2θ°] (±0.5, e.g., ±0.2)** | **Relative Intensity** |
|---|---|
| 9.7 | 98.27 |
| 10.2 | 100.00 |
| 11.3 | 22.47 |
| 14.0 | 5.01 |
| 14.6 | 12.36 |
| 19.9 | 5.63 |
| 21.1 | 3.72 |
| 22.2 | 5.37 |
| 22.7 | 4.04 |
| 24.5 | 2.99 |
| 25.3 | 17.65 |
| 27.2 | 3.10 |
| 32.4 | 5.29 |
| 36.7 | 2.72 |

In some embodiments, the crystalline form of sepiapterin free base is crystalline Form B of sepiapterin free base and has peaks at diffraction angle 2θ (°) of 8.4°±0.5, e.g., 8.4°±0.2, 16.9°±0.5, e.g., 16.9 °±0.2, and 25.4°±0.5, e.g., 25.4°±0.2 as measured by X-ray diffractometry by irradiation with Cu Kα X-rays or calculated from X-ray diffractometry. In some embodiments, the crystalline Form B of sepiapterin free base has peaks at diffraction angle 2θ (°) of 8.4°±0.5, e.g., 8.4°±0.2, 14.9°±0.5, e.g., 14.9°±0.2, 16.9°±0.5 ,e.g., 16.9°±0.2, 25.4°±0.5, e.g., 25.4°±0.2, and 34.1°±0.5, e.g., 34.1°±0.2 as measured by X-ray diffractometry by irradiation with Cu Kα X-rays or calculated from X-ray diffractometry. In an essentially pure material of this crystalline form, peaks can be observed at angles of refraction 2θ as set forth in Table 2. Alternatively, or in addition, this crystalline form is characterized by a DSC curve showing a melting event at 195.2° C.

**Table 2**

| **Position [2θ°] (±0.5, e.g., ±0.2)** | **Relative Intensity** |
|---|---|
| 8.4 | 100.00 |
| 14.9 | 2.34 |
| 16.9 | 10.70 |
| 25.4 | 84.90 |
| 34.1 | 3.00 |

In some embodiments, the crystalline form of sepiapterin free base is crystalline Form C of sepiapterin free base and has peaks at diffraction angle 2θ (°) of 5.7°±0.5, e.g., 5.7°±0.2, 7.8°±0.5, e.g., 7.8°±0.2, and 25.4°±0.5, e.g., 25.4°±0.2 as measured by X-ray diffractometry by irradiation with Cu Kα X-rays or calculated from X-ray diffractometry. In some embodiments, the crystalline Form C of sepiapterin free base has peaks at diffraction angle 2θ (°) of 5.7°±0.5, e.g., 5.7°±0.2, 7.8°±0.5, e.g., 7.8°±0.2, 9.1°±0.5, e.g., 9.1°±0.2, 11.5°±0.5, e.g., 11.5°±0.2, 15.3°±0.5, e.g., 15.3°±0.2, 16.0°±0.5, e.g., 16.0°±0.2, 20.1°±0.5, e.g., 20.1°±0.2, 25.4°±0.5, e.g., 25.4°±0.2, and 26.6°±0.5, e.g., 26.6°±0.2, as measured by X-ray diffractometry by irradiation with Cu Kα X-rays or calculated from X-ray diffractometry. In an essentially pure material of this crystalline form, peaks can be observed at angles of refraction 2θ as set forth in Table 3. Alternatively or in addition, this crystalline form is characterized by a DSC curve showing five endothermal peaks at 58.3° C, 101.8° C, 129.8° C, 156.5° C, and 168.3° C.

**Table 3**

| **Position [2θ°] (±0.5, e.g., ±0.2)** | **Relative Intensity** |
|---|---|
| 5.7 | 48.91 |
| 7.8 | 100.00 |
| 9.1 | 59.49 |
| 10.4 | 8.72 |
| 11.5 | 24.53 |
| 12.9 | 8.50 |
| 14.8 | 9.24 |
| 15.3 | 12.53 |
| 16.0 | 14.09 |
| 17.2 | 7.22 |
| 18.2 | 4.25 |
| 19.2 | 5.78 |
| 20.1 | 14.54 |
| 21.5 | 6.47 |
| 22.9 | 6.85 |
| 23.7 | 4.80 |
| 25.4 | 65.68 |
| 26.6 | 14.53 |
| 27.4 | 8.39 |
| 31.5 | 3.74 |
| 34.2 | 4.36 |

In some embodiments, the crystalline form of sepiapterin free base is crystalline Form D of sepiapterin free base and has peaks at diffraction angle 2θ (°) of 8.9°±0.5, e.g., 8.9°±0.2, 10.3°±0.5, e.g., 10.3°±0.2, and 26.0°±0.5, e.g., 26.0°±0.2, as measured by X-ray diffractometry by irradiation with Cu Kα X-rays or calculated from X-ray diffractometry. In some embodiments, the crystalline Form D of sepiapterin free base has peaks at diffraction angle 2θ (°) of 8.9°±0.5, e.g., 8.9°±0.2, 10.3°±0.5, e.g., 10.3°±0.2, 10.9°±0.5, e.g., 10.9 °±0.2, 17.8°±0.5, e.g., 17.8°±0.2, 24.9°±0.5, e.g., 24.9°±0.2, 26.0°±0.5, e.g., 26.0°±0.2, 26.7°±0.5, e.g., 26.7°±0.2, 26.8°±0.5, e.g., 26.8°±0.2, and 28.3°±0.5, e.g., 28.3°±0.2, as measured by X-ray diffractometry by irradiation with Cu Kα X-rays or calculated from X-ray diffractometry. In an essentially pure material of this crystalline form, peaks can be observed at angles of refraction 2θ as set forth in Table 4. Alternatively or in addition, this crystalline form is characterized by a DSC curve showing three endotherms at 42.7° C, 66.3° C, and 232.9° C.

**Table 4**

| **Position [2θ°] (±0.5, e.g., ±0.2)** | **Relative Intensity** |
|---|---|
| 8.9 | 100.00 |
| 10.3 | 49.92 |
| 10.9 | 19.96 |
| 11.6 | 2.15 |
| 13.6 | 2.99 |
| 14.2 | 3.45 |
| 14.8 | 2.35 |
| 15.4 | 2.59 |
| 16.4 | 1.55 |
| 17.2 | 2.33 |
| 17.8 | 6.24 |
| 19.6 | 2.62 |
| 20.1 | 2.28 |
| 20.5 | 3.09 |
| 20.8 | 2.27 |
| 21.3 | 3.60 |
| 22.3 | 4.79 |
| 23.7 | 4.31 |
| 24.9 | 5.19 |
| 26.0 | 41.94 |
| 26.7 | 8.58 |
| 26.8 | 9.17 |
| 27.4 | 3.98 |
| 28.3 | 4.75 |
| 28.7 | 6.60 |
| 29.8 | 3.03 |
| 31.8 | 2.72 |
| 33.0 | 2.03 |
| 35.5 | 1.57 |
| 37.1 | 1.09 |

In some embodiments, the crystalline form of sepiapterin free base is crystalline Form A of sepiapterin free base and has peaks at diffraction angle 2θ (°) of 4.7°±0.5, e.g., 4.7°±0.2, 7.4°±0.5, e.g., 7.4°±0.2, and 26.2°±0.5, e.g., 26.2°±0.2, as measured by X-ray diffractometry by irradiation with Cu Kα X-rays or calculated from X-ray diffractometry. In some embodiments, the crystalline Form A of sepiapterin free base has peaks at diffraction angle 2θ (°) of 4.7°±0.5, e.g., 4.7°±0.2, 7.4°±0.5, e.g., 7.4°±0.2, 9.5°±0.5, e.g., 9.5°±0.2, 11.3°±0.5, e.g., 11.3°±0.2, 15.6°±0.5, e.g., 15.6°±0.2, 16.4°±0.5, e.g., 16.4°±0.2, 26.2°±0.5, e.g., 26.2°±0.2, and 27.2°±0.5, e.g., 27.2°±0.2 as measured by X-ray diffractometry by irradiation with Cu Kα X-rays or calculated from X-ray diffractometry. In an essentially pure material of this crystalline form, peaks can be observed at angles of refraction 2θ as set forth in Table 5. Alternatively, or in addition, this crystalline form is characterized by a DSC curve showing endothermal peaks at 82.8° C and 179.8° C.

**Table 5**

| **Position [2θ°] (±0.5, e.g., ±0.2)** | | **Relative Intensity** |
|---|---|---|
| | 4.7 | 47.76 |
| | 7.4 | 100.00 |
| | 9.5 | 33.54 |
| | 11.3 | 19.31 |
| | 12.4 | 8.49 |
| | 13.4 | 3.60 |
| | 14.2 | 8.24 |
| | 15.6 | 15.08 |
| | 16.4 | 11.97 |
| | 17.6 | 8.35 |
| | 18.4 | 5.03 |
| | 19.8 | 9.18 |
| | 21.5 | 5.44 |
| | 24.4 | 5.56 |
| | 26.2 | 35.37 |
| | 27.2 | 19.11 |
| | 28.9 | 5.93 |

In some embodiments, the crystalline form of sepiapterin free base is crystalline Form E of sepiapterin free base and has at peaks at diffraction angle 2θ (°) of 6.0°±0.5, 6.0 °±0.2 10.6°±0.5, 10.6 °±0.2, 12.1°±0.5, e.g., 12.1°±0.2, 15.9°±0.5, e.g., 15.9°±0.2, 20.9°±0.5, e.g., 20.9°±0.2, and 24.6°±0.5, e.g., 24.6°±0.2, as measured by X-ray diffractometry by irradiation with Cu Kα X-rays or calculated from X-ray diffractometry. In some embodiments, the crystalline Form E of sepiapterin free base has peaks at diffraction angle 2θ (°) of 6.0°±0.5, e.g., 6.0°±0.2, 10.6°±0.5, e.g., 10.6°±0.2, 12.1°±0.5, e.g., 12.1°±0.2, 15.9°±0.5, e.g., 15.9°±0.2, 18.1°±0.5, e.g., 18.1°±0.2, 20.9°±0.5, e.g., 20.9°±0.2, 22.1°±0.5, e.g., 22.1°±0.2, 24.6°±0.5, e.g., 24.6°±0.2, 26.1°±0.5, e.g., 26.1°±0.2, 28.1°±0.5, e.g., 28.1°±0.2, 28.9°±0.5, e.g., 28.9°±0.2, 32.1°±0.5, e.g., 32.1°±0.2, and 37.0°±0.5, e.g., 37.0°±0.2 as measured by X-ray diffractometry by irradiation with Cu Kα X-rays or calculated from X-ray diffractometry. In an essentially pure form of this crystalline form, peaks can be observed at angles of refraction 2θ as set forth in Table 6. Alternatively or in addition, this crystalline form is characterized by a DSC curve showing two endothermal peaks at 112.9° C and 195.8° C.

**Table 6**

| **Position [2θ°] (±0.5, e.g., ±0.2)** | | **Relative Intensity** |
|---|---|---|
| | 6.0 | 100.00 |
| | 10.6 | 20.78 |
| | 12.1 | 31.95 |
| | 15.9 | 12.83 |
| | 18.1 | 3.39 |
| | 20.9 | 11.63 |
| | 22.1 | 2.79 |
| | 24.6 | 8.28 |
| | 26.1 | 0.88 |
| | 28.1 | 7.33 |
| | 28.9 | 3.77 |
| | 32.1 | 3.57 |
| | 37.0 | 1.03 |

In some embodiments, the crystalline form of sepiapterin free base is crystalline Form G of sepiapterin free base and has peaks at diffraction angle 2θ (°) of 10.0°±0.5, e.g., 10.0°±0.2, 10.6°±0.5, e.g., 10.6°±0.2, and 25.7°±0.5, e.g., 25.7°±0.2, as measured by X-ray diffractometry by irradiation with Cu Kα X-rays or calculated from X-ray diffractometry. In some embodiments, the Form G of sepiapterin free base has peaks at diffraction angle 2θ (°) of 10.0°±0.5, e.g., 10.0°±0.2, 10.6°±0.5, e.g., 10.6°±0.2, 11.2°±0.5, e.g., 11.2°±0.2, 15.3°±0.5, e.g., 15.3°±0.2, 15.9°±0.5, e.g., 15.9°±0.2, 22.8°±0.5, e.g., 22.8°±0.2, 24.4°±0.5, e.g., 24.4°±0.2, 25.0°±0.5, e.g., 25.0°±0.2, 25.7°±0.5, e.g., 25.7°±0.2, and 26.6°±0.5, e.g., 26.6°±0.2 as measured by X-ray diffractometry by irradiation with Cu Kα X-rays or calculated from X-ray diffractometry. In an essentially pure material of this crystalline form, peaks can be observed at angles of refraction 2θ as set forth in Table 7.

**Table 7**

| **Position [2θ°] (±0.5, e.g., ±0.2)** | **Relative Intensity** |
|---|---|
| 5.3 | 8.30 |
| 6.9 | 4.54 |
| 10.0 | 100.00 |
| 10.6 | 69.64 |
| 11.2 | 6.59 |
| 13.5 | 7.52 |
| 15.3 | 26.59 |
| 15.9 | 26.43 |
| 16.0 | 23.41 |
| 16.9 | 4.28 |
| 18.6 | 13.02 |
| 19.3 | 11.90 |
| 20.1 | 7.22 |
| 20.8 | 11.01 |
| 22.8 | 16.77 |
| 23.5 | 19.60 |
| 24.4 | 41.45 |
| 25.0 | 23.99 |
| 25.7 | 65.40 |
| 26.6 | 39.64 |
| 27.6 | 13.04 |
| 28.7 | 6.55 |
| 30.8 | 14.76 |
| 32.2 | 9.63 |
| 33.7 | 5.16 |
| 37.5 | 5.80 |

In some embodiments, the crystalline form of the hydrochloride salt of sepiapterin has peaks at diffraction angle 2θ (°) of 7.8°±0.5, e.g., 7.8°±0.2, 12.9°±0.5, e.g., 12.9°±0.2, and 26.2°±0.5, e.g., 26.2°±0.2, as measured by X-ray diffractometry by irradiation with Cu Kα X-rays or calculated from X-ray diffractometry. In some embodiments, the most intense peak in the X-ray diffraction diagram of the crystalline form of the hydrochloride salt of sepiapterin is observed at an angle of refraction 2θ of 7.8°±0.5, e.g., 7.8°±0.2.In an essentially pure material of this crystalline hydrochloride salt of sepiapterin, peaks can be observed at angles of refraction 2θ as set forth in Table 8. Alternatively or in addition, the crystalline hydrochloride salt of sepiapterin is characterized by a DSC curve showing an endotherm at 225.9° C.

**Table 8**

| **Position [2θ°] (±0.5, e.g., ±0.2)** | **Relative Intensity** |
|---|---|
| 7.8 | 100.00 |
| 8.9 | 6.89 |
| 12.9 | 58.56 |
| 15.6 | 8.52 |
| 17.9 | 25.23 |
| 19.2 | 5.48 |
| 21.1 | 10.97 |
| 23.6 | 25.15 |
| 25.2 | 22.66 |
| 26.2 | 45.91 |
| 27.6 | 32.94 |
| 30.3 | 10.50 |
| 31.7 | 7.83 |
| 34.2 | 8.87 |
| 36.7 | 3.67 |

In some embodiments, the crystalline Form 1 methanesulfonate salt of sepiapterin has peaks at diffraction angle 2θ (°) of 7.8°±0.5, e.g., 7.8°±0.2, 23.5°±0.5, e.g., 23.5°±0.2, and 29.0°±0.5, e.g., 29.0°±0.2 as measured by X-ray diffractometry by irradiation with Cu Kα X-rays or calculated from X-ray diffractometry. In some embodiments, the most intense peak in the X-ray diffraction diagram is observed at an angle of refraction 2θ of 23.5°±0.5, e.g., 23.5°±0.2. In an essentially pure material of the crystalline Form 1 methanesulfonate salt of sepiapterin, peaks can be observed at angles of refraction 2θ as set forth in Table 9. Alternatively or in addition, the crystalline form 1 methanesulfonate salt of sepiapterin is characterized by a DSC curve showing two endotherms at 186.0° C and 229.1° C.

**Table 9**

| **Position [2θ°] (±0.5, e.g., ±0.2)** | **Relative Intensity** |
|---|---|
| 7.9 | 21.77 |
| 11.7 | 8.20 |
| 13.7 | 8.52 |
| 15.7 | 4.79 |
| 16.6 | 5.34 |
| 18.0 | 5.66 |
| 19.8 | 2.10 |
| 20.3 | 5.36 |
| 20.9 | 2.43 |
| 22.3 | 4.25 |
| 22.7 | 2.15 |
| 23.5 | 100.00 |
| 24.7 | 3.69 |
| 25.6 | 2.70 |
| 26.8 | 1.79 |
| 27.2 | 1.68 |
| 28.3 | 2.75 |
| 29.0 | 57.60 |
| 29.8 | 5.18 |
| 30.5 | 1.37 |
| 32.2 | 4.66 |
| 33.0 | 1.64 |
| 36.5 | 1.29 |

In some embodiments, the crystalline Form 2 methanesulfonate salt of sepiapterin has peaks at diffraction angle 2θ (°) of 7.9°±0.5, e.g., 7.9°±0.2, 23.4°±0.5, e.g., 23.4°±0.2, and 28.9°±0.5, e.g., 28.9°±0.2 as measured by X-ray diffractometry by irradiation with Cu Kα X-rays or calculated from X-ray diffractometry. In some embodiments, the most intense peak in the X-ray diffraction diagram is observed at an angle of refraction 2θ of 7.9°±0.5, e.g., 7.9°±0.2. In an essentially pure material of the crystalline Form 2 methanesulfonate salt of sepiapterin, peaks can be observed at angles of refraction 2θ as set forth in Table 10. Alternatively or in addition, the crystalline form 2 methanesulfonate salt of sepiapterin is characterized by a DSC curve showing three endotherms at 75.5° C, 182.6° C, and 234.9° C.

**Table 10**

| **Position [2θ°] (±0.5, e.g., ±0.2)** | **Relative Intensity** |
|---|---|
| 7.9 | 100.00 |
| 11.0 | 21.32 |
| 12.1 | 22.02 |
| 13.5 | 79.87 |
| 15.7 | 11.87 |
| 17.8 | 9.81 |
| 19.7 | 10.93 |
| 21.3 | 26.79 |
| 23.4 | 96.13 |
| 24.1 | 24.88 |
| 24.3 | 22.10 |
| 25.5 | 9.45 |
| 26.0 | 11.27 |
| 27.6 | 7.63 |
| 28.9 | 95.64 |
| 31.2 | 4.39 |
| 36.1 | 6.65 |

In some embodiments, the crystalline Form 3 methanesulfonate salt of sepiapterin has peaks at diffraction angle 2θ (°) of 21.7°±0.5, e.g., 21.7°±0.2, 26.1°±0.5, e.g., 26.1°±0.2, and 28.9°±0.5, e.g., 28.9°±0.2 as measured by X-ray diffractometry by irradiation with Cu Kα X-rays or calculated from X-ray diffractometry. In some embodiments, the most intense peak in the X-ray diffraction diagram is observed at an angle of refraction 2θ of 26.1°±0.5, e.g., 26.1°±0.2. In an essentially pure material of the crystalline Form 3 methanesulfonate salt of sepiapterin, peaks can be observed at angles of refraction 2θ as set forth in Table 11. Alternatively or in addition, the crystalline form 3 methanesulfonate salt of sepiapterin is characterized by a DSC curve showing two endotherms at 195.1° C and 240.1° C.

**Table 11**

| **Position [2θ°] (±0.5, e.g., ±0.2)** | **Relative Intensity** |
|---|---|
| 8.2 | 47.29 |
| 10.8 | 56.14 |
| 12.6 | 16.34 |
| 13.2 | 15.90 |
| 14.0 | 24.39 |
| 15.0 | 12.03 |
| 15.9 | 16.20 |
| 18.2 | 22.97 |
| 20.1 | 25.53 |
| 20.5 | 14.97 |
| 21.3 | 22.70 |
| 21.7 | 71.48 |
| 22.2 | 11.40 |
| 23.6 | 46.37 |
| 24.8 | 44.00 |
| 25.5 | 9.08 |
| 26.1 | 100.00 |
| 27.3 | 3.52 |
| 28.9 | 68.42 |
| 31.2 | 4.49 |
| 32.1 | 6.48 |
| 34.8 | 5.95 |
| 35.6 | 1.67 |
| 39.1 | 2.91 |

In some embodiments, the crystalline nicotinate salt of sepiapterin has peaks at diffraction angle 2θ (°) of 9.5°±0.5, e.g., 9.5°±0.2, 9.9°±0.5, e.g., 9.9°±0.2, and 24.5°±0.5, e.g., 24.5°±0.2 as measured by X-ray diffractometry by irradiation with Cu Kα X-rays or calculated from X-ray diffractometry. In some embodiments, the most intense peak in the X-ray diffraction diagram is observed at an angle of refraction 2θ of 24.5°±0.5, e.g., 24.5°±0.2. In an essentially pure material of the crystalline nicotinate salt of sepiapterin, peaks can be observed at angles of refraction 2θ as set forth in Table 12. Alternatively or in addition, the crystalline nicotinate salt of sepiapterin is characterized by a DSC curve showing an endotherm at 221.9° C.

**Table 12**

| **Position [2θ°] (±0.5, e.g., ±0.2)** | **Relative Intensity** |
|---|---|
| 9.5 | 10.29 |
| 9.9 | 53.95 |
| 11.5 | 9.31 |
| 12.0 | 11.76 |
| 14.7 | 14.20 |
| 15.9 | 17.61 |
| 17.5 | 7.53 |
| 19.0 | 5.37 |
| 20.8 | 5.88 |
| 21.3 | 6.12 |
| 21.7 | 7.20 |
| 23.2 | 34.05 |
| 24.5 | 100.00 |
| 25.2 | 12.90 |
| 28.0 | 8.51 |
| 31.1 | 5.39 |
| 32.3 | 4.52 |
| 33.4 | 8.02 |
| 35.1 | 5.05 |

In some embodiments, the crystalline p-toluenesulfonate salt of has peaks at diffraction angle 2θ (°) of 6.5°±0.5, e.g., 6.5°±0.2, 15.1°±0.5, e.g., 15.1°±0.2, and 23.4°±0.5, e.g., 23.4°±0.2 as measured by X-ray diffractometry by irradiation with Cu Kα X-rays or calculated from X-ray diffractometry. In some embodiments, the most intense peak in the X-ray diffraction diagram is observed at an angle of refraction 2θ of 6.5°±0.5, e.g., 6.5°±0.2. In an essentially pure material of the p-toluenesulfonate salt of sepiapterin, peaks can be observed at angles of refraction 2θ as set forth in Table 13. Alternatively or in addition, the crystalline p-toluenesulfonate salt of sepiapterin is characterized by a DSC curve showing three endotherms at 77.2° C, 202.4° C and 260.2° C.

**Table 13**

| **Position [2θ°] (±0.5, e.g., ±0.2)** | **Relative Intensity** |
|---|---|
| 6.5 | 100.00 |
| 12.9 | 1.79 |
| 14.3 | 1.39 |
| 15.1 | 15.36 |
| 16.2 | 5.33 |
| 18.4 | 8.96 |
| 19.6 | 3.06 |
| 20.2 | 4.86 |
| 21.8 | 2.23 |
| 22.5 | 2.95 |
| 23.1 | 7.99 |
| 23.4 | 9.14 |
| 24.5 | 1.81 |
| 26.0 | 2.48 |
| 27.0 | 4.49 |
| 27.3 | 3.93 |
| 28.1 | 5.31 |
| 28.4 | 5.59 |
| 28.8 | 2.05 |
| 30.6 | 2.24 |
| 31.0 | 1.98 |
| 32.6 | 1.82 |

In some embodiments, the crystalline benzenesulfonate salt of sepiapterin has peaks at diffraction angle 2θ (°) of 6.5°±0.5, e.g., 6.5°±0.2, 14.8°±0.5, e.g., 14.8°±0.2, and 19.6°±0.5, e.g., 19.6°±0.2 as measured by X-ray diffractometry by irradiation with Cu Kα X-rays or calculated from X-ray diffractometry. In some embodiments, the most intense peak in the X-ray diffraction diagram is observed at an angle of refraction 2θ of 6.5°±0.5, e.g., 6.5°±0.2. In an essentially pure material of the benzenesulfonate salt of sepiapterin, peaks can be observed at angles of refraction 2θ as set forth in Table 14. Alternatively or in addition, the crystalline benzenesulfonate salt of sepiapterin is characterized by a DSC curve showing two endotherms at 202.3° C and 265.5° C.

**Table 14**

| **Position [2θ°] (±0.5, e.g., ±0.2)** | **Relative Intensity** |
|---|---|
| 4.9 | 5.90 |
| 6.5 | 100.00 |
| 14.8 | 16.73 |
| 17.8 | 4.23 |
| 19.6 | 7.98 |
| 21.5 | 2.49 |
| 23.7 | 3.46 |
| 24.5 | 3.84 |
| 26.1 | 3.29 |

In some embodiments, the crystalline phosphate salt of sepiapterin has peaks at diffraction angle 2θ (°) of 16.6°±0.5, e.g., 16.6°±0.2, 22.2°±0.5, e.g., 22.2°±0.2, and 25.6°±0.5, e.g., 25.6°±0.2 as measured by X-ray diffractometry by irradiation with Cu Kα X-rays or calculated from X-ray diffractometry. In some embodiments, the most intense peak in the X-ray diffraction diagram is observed at an angle of refraction 2θ of 25.6°±0.5, e.g., 25.6°±0.2. In an essentially pure material of the crystalline phosphate salt of sepiapterin, peaks can be observed at angles of refraction 2θ as set forth in Table 15. Alternatively or in addition, the crystalline phosphate salt of sepiapterin is characterized by a DSC curve showing three endotherms at 125.9° C, 152.1° C, and 157.6° C.

**Table 15**

| **Position [2θ°] (±0.5, e.g., ±0.2)** | **Relative Intensity** |
|---|---|
| 5.5 | 4.41 |
| 8.1 | 1.21 |
| 8.9 | 2.21 |
| 10.3 | 1.79 |
| 10.8 | 5.80 |
| 15.3 | 1.84 |
| 16.6 | 8.35 |
| 17.7 | 1.95 |
| 20.3 | 1.40 |
| 21.2 | 1.61 |
| 22.2 | 9.77 |
| 23.1 | 1.74 |
| 25.6 | 100.00 |
| 30.8 | 6.31 |
| 31.1 | 4.85 |
| 33.5 | 0.73 |
| 36.0 | 1.70 |

In some embodiments, the crystalline malonate salt of sepiapterin has peaks at diffraction angle 2θ (°) of 6.9°±0.5, e.g., 6.9°±0.2, 22.7°±0.5, e.g., 22.7°±0.2 and 23.8°±0.5, e.g., 23.8°±0.2 as measured by X-ray diffractometry by irradiation with Cu Kα X-rays or calculated from X-ray diffractometry. In some embodiments, the most intense peak in the X-ray diffraction diagram is observed at an angle of refraction 2θ of 6.9°±0.5, e.g., 6.9°±0.2. In an essentially pure material of the crystalline malonate salt of sepiapterin, peaks can be observed at angles of refraction 2θ as set forth in Table 16. Alternatively or in addition, the crystalline malonate salt of sepiapterin is characterized by a DSC curve showing a melting event at 115.8° C.

**Table 16**

| **Position [2θ°] (±0.5, e.g., ±0.2)** | **Relative Intensity** |
|---|---|
| 6.9 | 100.00 |
| 8.4 | 13.11 |
| 10.6 | 7.62 |
| 16.4 | 5.63 |
| 17.8 | 9.73 |
| 19.3 | 8.96 |
| 20.1 | 9.99 |
| 22.2 | 10.50 |
| 22.7 | 20.52 |
| 23.8 | 34.02 |
| 24.5 | 5.82 |
| 25.5 | 24.50 |
| 26.6 | 4.00 |
| 27.3 | 6.96 |
| 29.8 | 5.38 |
| 33.1 | 12.08 |

In some embodiments, the crystalline L-tartrate salt of sepiapterin has peaks at diffraction angle 2θ (°) of 7.4°±0.5, e.g., 7.4°±0.2, 14.2°±0.5, e.g., 14.2°±0.2, and 21.8°±0.5, e.g., 21.8°±0.2 as measured by X-ray diffractometry by irradiation with Cu Kα X-rays or calculated from X-ray diffractometry. In some embodiments, the most intense peak in the X-ray diffraction diagram is observed at an angle of refraction 2θ of 7.4°±0.5, e.g., 7.4°±0.2. In an essentially pure material of the crystalline L-tartrate salt of sepiapterin, peaks can be observed at angles of refraction 2θ as set forth in Table 17. Alternatively or in addition, the crystalline L-tartrate salt of sepiapterin is characterized by a DSC curve showing two endotherms at 97.2° C and 160.6° C.

**Table 17**

| **Position [2θ°] (±0.5, e.g., ±0.2)** | **Relative Intensity** |
|---|---|
| 7.4 | 100.00 |
| 10.1 | 47.99 |
| 14.2 | 82.76 |
| 14.7 | 27.06 |
| 19.1 | 21.16 |
| 20.2 | 29.91 |
| 21.8 | 85.30 |
| 22.1 | 53.68 |
| 23.9 | 85.30 |
| 24.9 | 19.26 |
| 25.5 | 28.45 |
| 26.8 | 18.58 |
| 29.7 | 21.59 |
| 31.6 | 10.10 |
| 32.9 | 22.18 |

In some embodiments, the crystalline gentisate salt of sepiapterin has peaks at diffraction angle 2θ (°) of 7.1°±0.5, e.g., 7.1°±0.2, 8.7°±0.5, e.g., 8.7°±0.2, and 26.7°±0.5, e.g., 26.7°±0.2 as measured by X-ray diffractometry by irradiation with Cu Kα X-rays or calculated from X-ray diffractometry. In some embodiments the most intense peak in the X-ray diffraction diagram is observed at an angle of refraction 2θ of 7.1°±0.5, e.g., 7.1°±0.2. In an essentially pure material of the crystalline gentisate salt of sepiapterin, peaks can be observed at angles of refraction 2θ as set forth in Table 18. Alternatively or in addition, the crystalline gentisate salt of sepiapterin is characterized by a DSC curve showing three endotherms at 70.5° C, 128.2° C, and 184.7° C.

**Table 18**

| **Position [2θ°] (±0.5, e.g., ±0.2)** | **Relative Intensity** |
|---|---|
| 5.7 | 17.29 |
| 7.1 | 100.00 |
| 8.7 | 42.69 |
| 10.4 | 3.94 |
| 11.3 | 11.69 |
| 12.1 | 4.13 |
| 14.3 | 21.10 |
| 16.0 | 6.46 |
| 16.4 | 5.94 |
| 17.0 | 5.85 |
| 17.6 | 7.93 |
| 19.1 | 8.27 |
| 20.20 | 3.47 |
| 20.7 | 2.90 |
| 21.5 | 3.37 |
| 23.6 | 2.69 |
| 24.4 | 4.50 |
| 26.7 | 52.20 |
| 27.1 | 35.49 |
| 28.2 | 8.74 |
| 28.9 | 4.31 |
| 29.9 | 2.62 |
| 31.4 | 2.99 |
| 34.4 | 1.28 |
| 35.8 | 3.54 |
| 37.6 | 0.57 |

In some embodiments, the crystalline fumarate salt of sepiapterin has peaks at diffraction angle 2θ (°) of 11.4°±0.5, e.g., 11.4°±0.2, 24.0°±0.5, e.g., 24.0°±0.2, and 28.2°±0.5, e.g., 28.2°±0.2 as measured by X-ray diffractometry by irradiation with Cu Kα X-rays or calculated from X-ray diffractometry. In some embodiments, the most intense peak in the X-ray diffraction diagram is observed at an angle of refraction 2θ of at least 24.0°±0.5, e.g., 24.0°±0.2. In an essentially pure material of the crystalline fumarate salt of sepiapterin, peaks can be observed at angles of refraction 2θ as set forth in Table 19. Alternatively or in addition, the crystalline fumarate salt of sepiapterin is characterized by a DSC curve showing two endotherms at 114.3° C and 229.7° C.

**Table 19**

| **Position [2θ°] (±0.5, e.g., ±0.2)** | **Relative Intensity** |
|---|---|
| 6.1 | 6.43 |
| 7.7 | 5.40 |
| 11.4 | 53.62 |
| 11.9 | 33.37 |
| 14.2 | 8.03 |
| 16.5 | 6.70 |
| 18.3 | 13.86 |
| 19.0 | 6.68 |
| 20.7 | 10.02 |
| 21.3 | 7.02 |
| 22.8 | 24.68 |
| 24.0 | 100.00 |
| 28.3 | 33.26 |
| 32.7 | 6.35 |
| 36.0 | 3.28 |
| 38.5 | 6.02 |
| | |

In some embodiments, the crystalline glycolate salt of has peaks at diffraction angle 2θ (°) of 7.6°±0.5, e.g., 7.6°±0.2, 10.7°±0.5, e.g., 10.7°±0.2, and 24.0°±0.5, e.g., 24.0°±0.2 as measured by X-ray diffractometry by irradiation with Cu Kα X-rays or calculated from X-ray diffractometry. In some embodiments, the most intense peak in the X-ray diffraction diagram is observed at an angle of refraction 2θ of 7.6°±0.5, e.g., 7.6°±0.2. In an essentially pure material of the crystalline glycolate salt of sepiapterin, peaks can be observed at angles of refraction 2θ as set forth in Table 20. Alternatively or in addition, the crystalline glycolate salt of sepiapterin is characterized by a DSC curve showing two endotherms at 133.9° C and 147.7° C.

**Table 20**

| **Position [2θ°] (±0.5, e.g., ±0.2)** | **Relative Intensity** |
|---|---|
| 4.8 | 6.23 |
| 7.6 | 100.00 |
| 10.3 | 68.06 |
| 10.7 | 70.69 |
| 15.3 | 36.51 |
| 18.2 | 24.25 |
| 18.7 | 27.26 |
| 19.9 | 2.66 |
| 21.2 | 17.11 |
| 24.0 | 96.62 |
| 24.4 | 18.44 |
| 28.8 | 47.57 |
| 30.3 | 7.43 |
| 32.5 | 4.42 |
| 33.3 | 7.49 |
| 34.3 | 5.21 |
| 36.3 | 7.37 |

In some embodiments, the crystalline acetate salt of has peaks at diffraction angle 2θ (°) of 6.2°±0.5, e.g., 6.2°±0.2, 12.0°±0.5, e.g., 12.0°±0.2, and 18.1°±0.5, e.g., 18.1°±0.2 as measured by X-ray diffractometry by irradiation with Cu Kα X-rays or calculated from X-ray diffractometry. In some embodiments, the most intense peak in the X-ray diffraction diagram is observed at an angle of refraction 2θ of at least 6.2°±0.5, e.g., 6.2°±0.2. In an essentially pure material of the crystalline acetate salt of sepiapterin, peaks can be observed at angles of refraction 2θ as set forth in Table 21. Alternatively or in addition, the crystalline acetate salt of sepiapterin is characterized by a DSC curve showing two endotherms at 146.1° C and 175.4° C.

**Table 21**

| **Position [2θ°] (±0.5, e.g., ±0.2)** | **Relative Intensity** |
|---|---|
| 6.2 | 100.00 |
| 10.2 | 23.29 |
| 12.0 | 71.59 |
| 18.1 | 31.27 |
| 21.1 | 20.29 |
| 24.2 | 14.92 |
| 25.2 | 23.03 |
| 27.3 | 13.30 |
| 29.1 | 12.95 |

In some embodiments, the crystalline Form 1 sulfate salt of sepiapterin has peaks at diffraction angle 2θ (°) of 5.1°±0.5, e.g., 5.1°±0.2, 7.8°±0.5, e.g., 7.8°±0.2, and 23.0°±0.5, e.g., 23.0°±0.2 as measured by X-ray diffractometry by irradiation with Cu Kα X-rays or calculated from X-ray diffractometry. In some embodiments, the most intense peak in the X-ray diffraction diagram is observed at an angle of refraction 2θ of 5.1°±0.5, e.g., 5.1°±0.2. In an essentially pure material of the crystalline Form 1 sulfate salt of sepiapterin, peaks can be observed at angles of refraction 2θ as set forth in Table 22. Alternatively or in addition, the crystalline form 1 sulfate salt of sepiapterin is characterized by a DSC curve showing three endotherms at 94.5° C, 158.3° C, and 209.9° C.

**Table 22**

| **Position [2θ°] (±0.5, e.g., ±0.2)** | **Relative Intensity** |
|---|---|
| 5.1 | 100.00 |
| 6.8 | 3.33 |
| 7.8 | 43.48 |
| 10.2 | 15.92 |
| 15.7 | 18.13 |
| 17.2 | 8.33 |
| 18.7 | 6.49 |
| 19.8 | 5.19 |
| 21.3 | 5.52 |
| 23.0 | 19.05 |
| 23.5 | 8.29 |
| 24.2 | 5.59 |
| 24.8 | 17.44 |
| 25.7 | 4.97 |
| 26.7 | 10.38 |
| 28.7 | 11.49 |
| 30.4 | 2.88 |
| 31.0 | 3.67 |

In some embodiments, the crystalline Form 2 sulfate salt of sepiapterin has peaks at diffraction angle 2θ (°) of 7.8°±0.5, e.g., 7.8°±0.2, 8.8°±0.5, e.g., 8.8°±0.2, and 24.1°±0.5, e.g., 24.1°±0.2 as measured by X-ray diffractometry by irradiation with Cu Kα X-rays or calculated from X-ray diffractometry. In some embodiments, the most intense peak in the X-ray diffraction diagram is observed at an angle of refraction 2θ of 8.8°±0.5, e.g., 8.8°±0.2. In an essentially pure material of the crystalline Form 2 sulfate salt of sepiapterin, peaks can be observed at angles of refraction 2θ as set forth in Table 23.

**Table 23**

| **Position [2θ°] (±0.5, e.g., ±0.2)** | **Relative Intensity** |
|---|---|
| 5.0 | 4.71 |
| 7.9 | 72.24 |
| 8.8 | 100.00 |
| 14.5 | 19.26 |
| 15.7 | 59.40 |
| 16.1 | 8.69 |
| 17.2 | 14.82 |
| 17.7 | 10.89 |
| 19.3 | 9.92 |
| 20.2 | 9.60 |
| 23.7 | 15.38 |
| 24.2 | 43.88 |
| 25.0 | 11.44 |
| 26.8 | 16.81 |
| 28.7 | 16.07 |
| 29.4 | 13.84 |
| 31.3 | 17.14 |
| 31.7 | 7.26 |
| 35.7 | 5.75 |

The present invention may employ a pharmaceutical composition including a pharmaceutically acceptable excipient and an effective amount of sepiapterin, or a pharmaceutically acceptable salt thereof. Examples of pharmaceutical compositions of sepiapterin and salts thereof can be found in WO 2019/046849 and WO 2019/232120.

The pharmaceutically acceptable excipient can be any of those conventionally used and is limited only by chemico-physical considerations, such as solubility and by the route of administration. It will be appreciated by one of skill in the art that, in addition to the following described pharmaceutical compositions, sepiapterin can be formulated as inclusion complexes, such as cyclodextrin inclusion complexes, or liposomes.

The pharmaceutically acceptable excipients described herein, for example, vehicles, adjuvants, excipients, or diluents, are well known to those who are skilled in the art and are readily available to the public. It is preferred that the pharmaceutically acceptable excipient be one which is chemically inert to the sepiapterin and one which has no detrimental side effects or toxicity under the conditions of use.

### Formulations which increase gastric and/or anterior intestine residence time

Gastro-retentive drug delivery is an approach with the drug formulation is designed to remain in the stomach longer, e.g., until drug release is complete.

Bioadhesive dosage forms utilize polymers that are capable of adhering to surfaces and result in a controlled release of the drug. The bioadhesive polymers may be anionic (e.g., carboxymethylcellulose, alginic acid, polyacrylic acid, pectin, carrageenan, polycarbophil, or carbomer); cationic (e.g., chitosan, polylysine, or polybrene); or non-ionic (e.g., polyethylene glycol, polyvinylpyrrolidone, dextran, or hydroxypropylmethylcellulose).

High-density dosage forms are designed to sit in the stomach at a lower level than the pyloric sphincter, and thus avoid emptying. Excipients suitable for high-density dosage forms include iron powder, barium sulphate, zinc oxide, and titanium oxide.

Expandable dosage forms are designed to expand in the stomach to be larger than the pyloric sphincter, and thus avoid emptying. For example, dosage forms including a drug core, a swellable hydrocolloid, and an outer semi-permeable polymer are suitable for expandable dosage forms.

Super-porous hydrogel dosage forms are designed, similarly to expandable dosage forms, to expand in the stomach to be larger than the pyloric sphincter. Super-porous hydrogel dosage forms may include polymers such as cross-carmellose sodium.

Floating dosage forms are designed to have a lower density than gastric fluid. Floating dosage forms may include compositions including ion exchange resin, a raft system, an inflatable chamber, an effervescent mixture, a swellable hydrocolloid, or a multiparticulate system.

### Antioxidants

Sepiapterin is prone to rapid oxidation when exposed to air. Accordingly, pharmaceutical compositions of the invention may include antioxidants. The antioxidant may minimize the oxidative degradation of sepiapterin. Examples of antioxidants include, but are not limited to, 4-chloro-2,6-di-tert-butylphenol, tocopherol, alpha-tocopherol, alkylated diphenylamines, ascorbic acid, ascorbyl myristate, ascorbyl palmitate, ascorbyl stearate, beta-carotene, butylated hydroxyanisole, butylated hydroxytoluene, citric acid, cysteine, D-alpha-tocopheryl polyethylene glycol 1000 succinate, deferoxamine methanesulfonate, dodecyl gallate, ethylenediaminetetraacetic acid, ethylparaben, folic acid, fumaric acid, gallic acid, glutathione, lecithin, malic acid, methylparaben, monothioglycerol, N-acetyl cysteine, nordihydroguaiaretic acid, octyl gallate, p-phenylenediamine, potassium ascorbate, potassium metabisulfite, potassium sorbate, propionic acid, propyl gallate, retinol, sorbic acid, sodium ascorbate, sodium bisulfite, sodium hydrosulfite, sodium isoascorbate, sodium metabisulfite, sodium sulfite, sodium thiosulfate, tartaric acid, tert-butylhydroquinone, thiourea, tocopheryl acetate, vitamin A, vitamin B6, vitamin B12, or vitamin E. Examples of antioxidants include, but are not limited to, ascorbic acid, tocopherol, retinol, ascorbyl palmitate, N-acetyl cysteine, glutathione, ethylenediaminetetraacetic acid, sodium bisulfite, sodium metabisulfite, thiourea, butylatedhydroxytoluene, butylatedhydroxyanisole, and vitamin E. In some embodiments, the pharmaceutical compositions of the invention include ascorbic acid, tocopherol, retinol, ascorbyl palmitate, N-acetyl cysteine, glutathione, butylatedhydroxytoluene, and/or butylatedhydroxyanisole as antioxidant.

In some embodiments, the pharmaceutical composition includes less than 10% antioxidant by weight, e.g., less than 9%, less than 8%, less than 7%, less than 6%, less than 5%, less than 4%, less than 3%, less than 2%, less than 1%, or substantially free of antioxidant. In some embodiments, the pharmaceutical composition includes 2-9% antioxidant by total weight, e.g., 2-4%, 3-5%, 4-6%, 5-7%, 6-8%, or 7-9%. In some embodiments, the pharmaceutical composition comprises 5-100% of the USP maximum daily dose of the antioxidant, e.g., in some embodiments, the pharmaceutical composition comprises about 5%, 10%, 15%, 20%, 30%, 40%, 50%, 60%, 70%, 80%, 90%, or 100% of the USP maximum daily dose of the antioxidant. In some embodiments, the ratio of sepiapterin to antioxidant is at least 1:1, e.g., 2:1, 3:1, 4:1, 5:1, 6:1, 7:1, 8:1, 9:1, or 10:1 by weight.

### Dispersants

In some embodiments, the pharmaceutical compositions of the invention include at least one dispersant. The dispersant may cause particles in the formulation to separate, e.g., release their medicinal substances on contact with moisture. Examples of dispersants include, but are not limited to, crosslinked polyvinylpyrrolidone, carboxymethylcellulose (e.g., croscarmellose salt, e.g., croscarmellose sodium), starch (e.g., sodium starch glycolate), or alginic acid. In some embodiments, the dispersant in the pharmaceutical composition is a carboxymethylcellulose such as a pharmaceutically acceptable salt of croscarmellose. In some embodiments, the pharmaceutical composition may include 0.1-1.5% dispersant by total weight, e.g., about 0.1%, 0.5%, 1%, or 1.5%. In some embodiments, the pharmaceutical composition includes less than 1.5% dispersant, e.g., less than 1%, less than 0.5%, or less than 0.1% by total weight.

### Anti-caking agents

In some embodiments, the pharmaceutical compositions of the invention include at least one anti-caking agent. In some embodiments, the pharmaceutical compositions include at least two anti-caking agents. Exemplary anti-caking agents include colloidal silicon dioxide, microcrystalline cellulose, tricalcium phosphate, microcrystalline cellulose, magnesium stearate, sodium bicarbonate, sodium ferrocyanide, potassium ferrocyanide, calcium ferrocyanide, calcium phosphate, sodium silicate, colloidal silicon dioxide, calcium silicate, magnesium trisilicate, talcum powder, sodium aluminosilicate, potassium aluminum silicate, calcium aluminosilicate, bentonite, aluminum silicate, stearic acid, and polydimethylsiloxane. In some embodiments, the at least one anti-caking agent is colloidal silicon dioxide or microcrystalline cellulose. In some embodiments, the pharmaceutical composition may include 65-75% anti-caking agent by total weight, e.g., about 65%, 67%, 70%, 73%, or 75%. In some embodiments, the pharmaceutical composition includes both colloidal silicon dioxide and microcrystalline cellulose. In some embodiments, the pharmaceutical composition includes 60-65% microcrystalline cellulose by total weight and 5-7% colloidal silicon dioxide by total weight.

### Dosing vehicle

In some embodiments, the pharmaceutical compositions of the invention are combined with a dosing vehicle prior to administration, e.g., a dosing vehicle with a viscosity of approximately 50-1750 centipoise (cP). One type of suspending agent that can be used is a combination of glycerin and sucrose in water (e.g., MEDISCA^{®} oral mix with 2.5% glycerin and 27% sucrose in water). An appropriate quantity of composition can be added to the dosing vehicle mixture and agitated to suspend the composition just prior to administration.

Other suspending agents may also be used as a dosing vehicle. Exemplary suspending agents include water, agar, alginic acid, sodium carboxymethyl cellulose, carrageenan, dextrin, gelatin, guar gum, hydroxyethyl cellulose, hydroxypropyl cellulose, hypromellose, methyl cellulose, polyethylene glycol, povidone, tragacanth, xanthan gum, or other suspending agents known in the art.

### Dosage

Sepiapterin, or pharmaceutically acceptable salt thereof, can be used in any suitable dose. Suitable doses and dosage regimens can be determined by conventional range finding techniques. Generally treatment is initiated with smaller dosages, which are less than the optimum dose. Thereafter, the dosage is increased by small increments until optimum effect under the circumstances is reached. For convenience, the total daily dosage may be divided and administered in portions during the day if desired. In proper doses and with suitable administration of certain compounds, the present invention provides for a wide range of responses. Typically, the dosages range from about 1 to about 150 mg/kg, or about 2.5 to about 150 mg/kg body weight of the subject being treated/day, e.g., 60 mg/kg/day. For example, in embodiments, sepiapterin, or pharmaceutically acceptable salt thereof, may be administered from about 10 mg/kg to about 150 mg/kg, from about 20 mg/kg to about 150 mg/kg, from about 10 mg/kg to about 60 mg/kg, from about 10 mg/kg to about 60 mg/kg, from about 20 mg/kg to about 60 mg/kg, from about 40 mg/kg to about 100 mg/kg, from about 100 mg/kg to about 150 mg/kg, from about 60 mg/kg to about 120 mg/kg, from about 80 mg/kg to about 100 mg/kg, from about 40 mg/kg to about 60 mg/kg, from about 2.5 mg/kg to about 20 mg/kg, from about 2.5 mg/kg to about 10 mg/kg, or from about 2.5 mg/kg to about 5 mg/kg, of subject body weight per day, one or more times a day, to obtain the desired therapeutic effect.

In some embodiments, the sepiapterin, or pharmaceutically acceptable salt thereof, can be formulated into unit solid oral dosage forms such as particles. In these embodiments, each unit solid oral dosage form, e.g., sachet, can comprise any suitable amount of the sepiapterin, or pharmaceutically acceptable salt thereof. For example, each solid oral dosage form can comprise about 2.5 mg, about 5 mg, about 10 mg, about 20 mg, about 30 mg, about 40 mg, about 50 mg, about 60 mg, about 70 mg, about 80 mg, about 90 mg, about 100 mg, about 125 mg, about 150 mg, about 175 mg, about 200 mg, about 225 mg, about 250 mg, about 275 mg, about 300 mg, about 325 mg, about 350 mg, about 375 mg, about 400 mg, about 425 mg, about 450 mg, about 475 mg, about 500 mg, about 750 mg, about 1 g, about 1.25 g, or about 1.5 g.

Sepiapterin, or a pharmaceutically acceptable salt thereof, can be used in the preparation of liquid formulations, such as in the form of a solution, suspension, or emulsion. Formulations suitable for oral administration include, but are not limited to, (a) capsules, sachets, tablets, lozenges, and troches, each containing a predetermined amount of the active ingredient, as solids or granules; (b) powders; (c) liquid solutions, such as an effective amount of the compound dissolved in diluents, such as water, saline, or orange juice; (d) suspensions in an appropriate liquid; and (e) suitable emulsions. Preferred are solid oral dosage forms such as capsule forms, tablet forms, and powder forms. Capsule forms can be of the ordinary hard- or soft-shelled gelatin type containing, for example, surfactants, lubricants, and inert fillers, such as lactose, sucrose, calcium phosphate, and cornstarch. Tablet forms can include one or more of lactose, sucrose, mannitol, corn starch, potato starch, alginic acid, microcrystalline cellulose, acacia, gelatin, guar gum, colloidal silicon dioxide, croscarmellose sodium, talc, magnesium stearate, calcium stearate, zinc stearate, stearic acid, and other excipients, colorants, diluents, buffering agents, disintegrating agents, moistening agents, preservatives, flavoring agents, and pharmacologically compatible excipients. Lozenge forms can comprise the active ingredient in a flavor, usually sucrose and acacia or tragacanth, as well as pastilles comprising the active ingredient in an inert base, such as gelatin and glycerin, or sucrose and acacia, emulsions, gels, and the like containing, in addition to the active ingredient, such excipients as are known in the art.

Formulations suitable for oral and/or parenteral administration include aqueous and non-aqueous, isotonic sterile injection solutions, which can contain anti-oxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The compound can be administered in a physiologically acceptable diluent in a pharmaceutical excipient, such as a sterile liquid or mixture of liquids, including water, saline, aqueous dextrose and related sugar solutions, an alcohol, such as ethanol, benzyl alcohol, or hexadecyl alcohol, glycols, such as propylene glycol or polyethylene glycol and other polyethylene alcohols, glycerol ketals, such as 2,2-dimethyl-1,3-dioxolane-4-methanol, ethers, such as poly(ethylene glycol) 400, an oil, a fatty acid, a fatty acid ester or glyceride, or an acetylated fatty acid glyceride with or without the addition of a pharmaceutically acceptable surfactant, such as a soap or a detergent, suspending agent, such as pectin, carbomers, methylcellulose, hydroxypropylmethylcellulose, or carboxymethylcellulose, or emulsifying agents and other pharmaceutical adjuvants.

The present invention features pharmaceutical compositions in an orally tolerable formula that contains a therapeutically effective amount of sepiapterin and less than 10% antioxidant. In some embodiments, the pharmaceutical composition is a granular formulation that is dispersed in a pharmaceutically acceptable excipient, for example the composition can be mixed into water and ingested by a subject (e.g., over the course of 5 to 10 minutes). Suitable formulations for use in the present invention are found in Remington's Pharmaceutical Sciences, Mack Publishing Company, Philadelphia, PA 22nd ed., 2010. Except insofar as any conventional excipient is incompatible with the active ingredient, its use in the pharmaceutical compositions is contemplated. Moreover, for animal (e.g., human) administration, it will be understood that preparations should meet sterility, pyrogenicity, general safety and purity standards as required by FDA Office of Biological Standards.

### Solid dosage form for oral administration

Formulations for oral use include particles containing the active ingredient(s) in a mixture with non-toxic pharmaceutically acceptable excipients, and such formulations are known to the skilled artisan (e.g., U.S. Patent Nos.: 5,817,307, 5,824,300, 5,830,456, 5,846,526, 5,882,640, 5,910,304, 6,036,949, 6,036,949, 6,372,218). Excipients may be, for example, inert diluents or fillers (e.g., sucrose, sorbitol, sugar, mannitol, microcrystalline cellulose, starches including potato starch, calcium carbonate, sodium chloride, lactose, calcium phosphate, calcium sulfate, or sodium phosphate); granulating and disintegrating agents (e.g., cellulose derivatives including microcrystalline cellulose, starches including potato starch, croscarmellose sodium, alginates, or alginic acid); binding agents (e.g., sucrose, glucose, sorbitol, acacia, alginic acid, sodium alginate, gelatin, starch, pregelatinized starch, microcrystalline cellulose, magnesium aluminum silicate, carboxymethylcellulose sodium, methylcellulose, hydroxypropyl methylcellulose, ethylcellulose, polyvinylpyrrolidone, or polyethylene glycol); and lubricating agents, glidants, anti-adhesives (e.g., magnesium stearate, zinc stearate, stearic acid, silicas, hydrogenated vegetable oils, or talc), and anti-caking agents (e.g., colloidal silicon dioxide, microcrystalline cellulose, tricalcium phosphate, microcrystalline cellulose, magnesium stearate, sodium bicarbonate, sodium ferrocyanide, potassium ferrocyanide, calcium ferrocyanide, calcium phosphate, sodium silicate, colloidal silicon dioxide, calcium silicate, magnesium trisilicate, talcum powder, sodium aluminosilicate, potassium aluminum silicate, calcium aluminosilicate, bentonite, aluminum silicate, stearic acid, polydimethylsiloxane). Other pharmaceutically acceptable excipients can be colorants, flavoring agents, plasticizers, humectants, and buffering agents. In some embodiments, excipients (e.g., flavoring agents) are packaged with the composition. In some embodiments, excipients (e.g., flavorings) are packaged separately from the composition (e.g., are combined with the composition prior to administration).

The solid compositions of the invention may include a coating adapted to protect the composition from unwanted chemical changes, (e.g., chemical degradation prior to the release of the active substances). The coating may be applied on the solid dosage form in a similar manner as that described in *Encyclopedia of Pharmaceutical Technology,* supra.

Powders and granulates may be prepared using the ingredients mentioned above in a conventional manner using, e.g., a mixer, a fluid bed apparatus, melt congeal apparatus, rotor granulator, extrusion/spheronizer, or spray drying equipment.

### Methods of Treatment

Any references herein to methods of treatment by therapy or surgery or in vivo diagnosis are to be interpreted as references to compounds, pharmaceutical compositions and medicaments described herein for use in those methods.

Sepiapterin, or a pharmaceutically acceptable salt thereof, serves as a useful therapeutic for treatment of glioblastoma. In the method of the present invention, the method further includes treatment with a chemotherapeutic agent (TMZ) and/or therapeutic radiation. In some embodiments of any of the methods described herein, the method further comprises therapeutic radiation treatment. In some embodiments of any of the methods described herein, the method further comprises administration of a chemotherapeutic agent (temozolomide). Thus, the various forms of sepiapterin, or a pharmaceutically acceptable salt thereof, in accordance with the present invention can be administered to a subject in an effective amount to obtain a treatment or amelioration of the disease, disorder or condition.

In some embodiments of any of the methods described herein, the method further comprises therapeutic radiation. In some embodiments of any of the methods described herein, the amount of radiation delivered during a treatment cycle may be at least about 1 Gy, at least about 2 Gy, at least about 5 Gy, at least about 10 Gy, at least about 20 Gy, at least about 30 Gy, at least about 40 Gy, at least about 50 Gy, at least about 60 Gy, at least about 70 Gy, at least about 80 Gy, at least about 90 Gy, at least about 100 Gy, at least about 150 Gy, at least about 200 Gy, at least about 300 Gy, at least about 400 Gy, or at least about 500 Gy. In various embodiments for each treatment cycle the radiation dosage is divided into more than one fraction, for example, 2, 3, 4, 5, 6, 7, 8, 9, 10, 20, 30, 40, or 50 or more fractions. In various embodiments a treatment cycle may be at least about 1 week, 2 weeks, 3 weeks, 4 weeks, 5 weeks, 6 weeks, 7 weeks, 8 weeks, or at least about 10 weeks. In various embodiments, one or more treatment cycles may be utilized (e.g. 2, 3, 4, 5, or more treatment cycles).

In some embodiments of any of the methods described herein, sepiapterin, or pharmaceutical salt thereof, is administered prior to the therapeutic radiation. In some embodiments of any of the methods described herein, the sepiapterin, or pharmaceutical salt thereof, is administered at least 1, 2, 3, 4, 5, 6, 7, 10, or more days prior to the therapeutic radiation.

In some embodiments of any of the methods described herein, sepiapterin, or pharmaceutical salt thereof, is administered subsequent to the therapeutic radiation. In some embodiments of any of the methods described herein, the sepiapterin, or pharmaceutical salt thereof, is administered at least 1, 2, 3, 4, 5, 6, 7, 10, 20, 30, 60, 90, 120, 150, 180, 210, 300, or more days subsequent to the therapeutic radiation.

In some embodiments of any of the methods described herein, the method further comprises administration of a chemotherapeutic agent, wherein the chemotherapeutic agent is temozolomide (TMZ). In various embodiments the dosage of TMZ is about 1 to about 1000 mg/m² per day, as measured by the subject body's surface area. In various embodiments, the dosage of TMZ is about 1 to about 500 mg/m² per day, or about 1 to about 250 mg/m² per day, or about 1 to about 100 mg/m² per day, or about 10 to about 500 mg/m² per day, or about 50 to about 250 mg/m² per day, or about 50 to about 100 mg/m² per day. The chemotherapeutic agent (TMZ) may be administered for at least about 1 week, 2 weeks, 3 weeks, 4 weeks, 5 weeks, 6 weeks, 7 weeks, 8 weeks, 9 weeks, 10 weeks. In various embodiments, the chemotherapeutic agent (TMZ) is administered prior to, concurrently with, and/or after administration of the sepiapterin, or pharmaceutical salt thereof. In various embodiments, the chemotherapeutic agent (TMZ) is administered prior to, concurrently with, and/or after administration of the therapeutic radiation treatment. In addition, the chemotherapeutic agent (TMZ) may be administered in a maintenance cycle, wherein the chemotherapeutic agent (TMZ) is administered for at least 1 day during a e.g. 28 day treatment cycle, for example, at least 2, 3, 4, 5, 6, 7, 8, 9, or 10 days during a 28 day maintenance treatment cycle.

The sepiapterin, or pharmaceutical salt thereof, may be administered to the subject before, during, and/or after the therapeutic radiation and/or the administration of the chemotherapeutic agent. In various embodiments of any of the methods described herein, the sepiapterin, or pharmaceutical salt thereof, is administered for at least about 1, 2, 3, 5, 6, 10, 14, 21, 28, or 30 days prior to initiation of radiation treatment. In various embodiments of any of the methods described herein, the sepiapterin. or pharmaceutical salt thereof, is administered continuously (e.g. daily) throughout radiation treatment. In various embodiments of any of the methods described herein, the sepiapterin, or pharmaceutical salt thereof, is administered for at least about 1, 2, 3, 5, 6, 10, 14, 21, 28, 30, 60, 90, 120, 150, 180, 210, 240 or more days subsequent to the radiation treatment. In various embodiments of any of the methods described herein, the sepiapterin, or pharmaceutical salt thereof, is administered for at least about 1, 2, 3, 5, 6, 10, 14, 21, 28, or 30 days prior to initiation of chemotherapeutic treatment (TMZ treatment). In various embodiments of any of the methods described herein, the sepiapterin, or pharmaceutical salt thereof, is administered continuously (e.g. daily) throughout chemotherapeutic treatment (TMZ treatment). In various embodiments of any of the methods described herein, the sepiapterin, or pharmaceutical salt thereof, is administered for at least about 1, 2, 3, 5, 6, 10, 14, 21, 28, 30, 60, 90, 120, 150, 180, 210, 240 or more days subsequent to the chemotherapeutic treatment (TMZ treatment).

In some embodiments of any of the methods described herein, the subject has the following treatment schedule: (1) an induction phase of sepiapterin, or pharmaceutical salt thereof, only (e.g. 1,2, 3, 4, 5, 6, 7, 8, 9, 10 or more days of sepiapterin, or pharmaceutical salt thereof, only); (2) a radiation phase of sepiapterin, or pharmaceutical salt thereof + chemotherapeutic agent (TMZ) + radiation (e.g. for 1, 2, 3, 4, 5, 6, 7, 8, 9, 10 or more weeks); (3) a chemotherapeutic "break" phase (e.g. sepiapterin or pharmaceutical salt thereof only, no chemotherapeutic agent (TMZ); (4) a maintenance phase, comprising one or more 28 day cycles, e.g. 2, 3, 4, 5, 6, 7, 8, or more cycles, in which sepiapterin, or pharmaceutical salt thereof, is administered continuously throughout the cycle(s), but wherein the chemotherapeutic agent (TMZ) is administered for only the first 1, 2, 3, 4, 5, 6, 7, 8, 9, 10 days of each 28 days cycle.

In some embodiments, the subject is a child (e.g., the subject is less than 18 years old, less than 17 years old, less than 16 years old, less than 15 years old, less than 14 years old, less than 13 years old, less than 12 years old, less than 11 years old, less than 10 years old, less than 9 years old, less than 8 years old, less than 7 years old, less than 6 years old, less than 5 years old, less than 4 years old, less than 3 years old, less than 2 years old, less than 1 year old). In some embodiments, the subject is an adult (e.g., the subject is greater than 18 years old). In some embodiments, the subject is at least 20 years old, at least 20 years old, at least 30 years old, at least 40 years old, at least 50 years old, at least 60 years old, at least 70 years old, at least 80 years old.

Sepiapterin, or a pharmaceutically acceptable salt thereof, may or may not be administered with food. Without being bound by theory, administration of sepiapterin with food results in an increase in plasma exposure of BH₄, e.g., by reducing the rate of absorption of sepiapterin. If the administered sepiapterin is absorbed quickly, e.g., by being administered on an empty stomach, sepiapterin reductase and/or dihydrofolate reductase in the cells may become saturated above Vₘₐₓ resulting in at least a portion of the administered sepiapterin leaving the cell without being reduced to 7,8-dihydrobiopterin and subsequently to BH₄. This excess sepiapterin may then be excreted without ever being converted to BH₄, resulting in lower levels of BH₄ in the plasma compared to administration of sepiapterin with food which reduces the rate of or prolongs the absorption of sepiapterin and results in reaction rates below, at or slightly above the Vₘₐₓ for substrate saturation of sepiapterin reductase enzyme and/or dihydrofolate reductase. Administration of sepiapterin, or a pharmaceutically acceptable salt thereof, with food unexpectedly results in an increase in the maximum BH₄ plasma concentration (Cmax) and the extent of exposure as measured by the area under the concentration time curve of time zero to last concentration (AUC₀₋ₗₐₛₜ) of BH₄ compared to administration without food. For example, the effective amount of sepiapterin, or a pharmaceutically acceptable salt thereof, is an amount (e.g., 1.0 mg/kg to 100 mg/kg per dose or 2.5 mg/kg to 100 mg/kg per dose) sufficient to produce a BH₄ concentration of at least 50 ng/mL (e.g., at least 60 ng/mL, at least 100 ng/mL, at least 200 ng/mL, at least 400 ng/mL, at least 600 ng/mL, at least 1000 ng/mL, or at least 2000 ng/mL, or from 50 ng/mL to 100 ng/mL, from 60 ng/mL to 400 ng/mL, from 200 ng/mL to 600 ng/mL, from 400 ng/mL to 1000 ng/mL, or from 600 ng/mL to 1500 ng/mL) in the plasma of the subject within 10 hours of administration with food. The effective amount may include a dose that is at least 5% (at least 10%, at least 20%, at least 50%, at least 70%, at least 90%, at least 100%, at least 110%, at least 120%, at least 130%, at least 140%, or at least 150%) lower than the dose sufficient to produce a maximum BH₄ plasma concentration (Cmax) of at least 50 ng/mL (e.g., at least 60 ng/mL, at least 100 ng/mL, at least 200 ng/mL, at least 400 ng/mL, at least 600 ng/mL, at least 1000 ng/mL, or at least 2000 ng/mL, or from 50 ng/mL to 100 ng/mL from 60 ng/mL to 400 ng/mL, from 200 ng/mL to 600 ng/mL, from 400 ng/mL to 1000 ng/mL, or from 600 ng/mL to 1500 ng/mL) in the plasma of the subject within 10 hours of administration of sepiapterin, or a pharmaceutically acceptable salt thereof, without food.

In some embodiments of any of the methods described herein, the food is a high protein food. In some embodiments of any of the methods described herein, the food is a high fat food (e.g., at least 25, 30, 40, or 50% of the calories are from fat). In some embodiments of any of the methods described herein, the food is a high protein and high fat food. In some embodiments, the food is high calorie food (e.g., the food includes at least 100 calories, e.g., at least 200 calories, at least 300 calories, at least 400 calories, at least 500 calories, e.g., 500-1500 or 800-1000 calories). In some embodiments of any of the methods described herein, the food is a meal, e.g., breakfast, lunch, or dinner. The sepiapterin, or a pharmaceutically acceptable salt thereof, may be provided in a separate composition from the consumed food (e.g., the sepiapterin or a pharmaceutically acceptable salt thereof, is not incorporated into a food product). In some embodiments of any of the methods described herein, the food is a low fat food.

Administration to the subject may occur less than 30 minutes prior to consuming food or after consuming food, e.g., immediately prior to the consumption of food up to 1 hour after consumption, such as substantially at the same time as food. The administration with food (e.g., occurring less than 30 minutes prior to consuming food or after consuming food, e.g., immediately prior to the consumption of food up to 1 hour after consumption) may result in an increase (e.g., at least 5%, at least 10%, at least 20%, at least 30%, at least 40%, at least 50%, at least 60%, at least 70%, at least 80%, at least 90%, at least 100%, at least 110%, at least 120%, at least 130%, at least 140%, or at least 150%) in the Cmax of BH4 or in the extent of production and resulting plasma exposure (AUC₀₋ₗₐₛₜ) of BH4 compared to administration without food (e.g., occurring more than 2 hours after consuming food until 30 minutes prior to consuming further food).

The sepiapterin or pharmaceutically acceptable salt thereof, may be administered to the subject without food, for example, more than 30 minutes prior to consuming food, or at least 2 hours after consuming food. In some embodiments in any of the methods described herein, administration occurs more than 30 minutes prior to consuming food, or at least 3 hours after consuming food. In some embodiments of any of the foregoing methods, the sepiapterin or salt pharmaceutically acceptable salt thereof, is administered without a high protein food. In some embodiments of any of the foregoing methods, the sepiapterin, or pharmaceutically acceptable salt thereof, is administered without a high fat food (e.g., at least 25, 30, 40, or 50% of the calories are from fat). In some embodiments of any of the foregoing methods the sepiapterin, or pharmaceutically acceptable salt thereof, is administered without a high protein and high fat food. In some embodiments, the sepiapterin, or pharmaceutically acceptable salt thereof, is administered without a high calorie food (e.g., the food includes at least 100 calories, e.g., at least 200 calories, at least 300 calories, at least 400 calories, at least 500 calories, e.g., 500-1500 or 800-1000 calories). In some embodiments of any of the foregoing methods, the sepiapterin, or pharmaceutically acceptable salt thereof, is administered without the food being a meal, e.g., breakfast, lunch, or dinner.

Without being bound by theory, administration of sepiapterin, or pharmaceutically acceptable salt thereof, without food may result in an increase in plasma, CSF, and/or brain exposure of sepiapterin by increasing the rate of absorption of sepiapterin. As sepiapterin passes through cell membranes efficiently, if the administered sepiapterin is absorbed quickly, e.g., by being administered on an empty stomach, the active transporters of sepiapterin and/or sepiapterin reductase enzymes in cells may be saturated resulting in at least a portion of the administered sepiapterin not entering the cells and/or leaving the cell without being reduced to 7,8-dihydrobiopterin. This excess sepiapterin in the plasma may then cross the blood brain barrier (BBB) and enter into brain cells prior to being converted to BH4, resulting in higher levels of BH4 in the brain (and/or CSF) compared to administration with food, which reduces the rate of absorption of sepiapterin and may not result in saturation of the sepiapterin transporters and intracellular sepiapterin reductase enzymes. Thus, administration of sepiapterin, or pharmaceutically acceptable salt thereof, without food unexpectedly results in an increase in the maximum plasma, CSF, and/or brain concentration (Cmax) and/or the extent of absorption (AUC₀₋ₗₐₛₜ) of sepiapterin compared to administration with food. The increased levels of sepiapterin in the plasma, CSF, and/or brain may be beneficial during treatment.

The actual dosage amount of a composition of the present invention administered to a subject can be determined by physical and physiological factors such as body weight, severity of condition, the type of disease being treated, previous or concurrent therapeutic interventions, idiopathy of the subject and on the route of administration. Depending upon the dosage and the route of administration, the number of administrations of a preferred dosage and/or an effective amount may vary according to the response of the subject. The practitioner responsible for administration will, in any event, determine the concentration of active ingredient(s) in a composition and appropriate dose(s) for the individual subject.

In some embodiments, subjects receive about 1 mg/kg to 120 mg/kg per dose (e.g., about 10 mg/kg to about 60 mg/kg, about 20 mg/kg to about 60 mg/kg, or about 20 mg/kg, about 30 mg/kg, about 40 mg/kg, about 50 mg/kg, about 60 mg/kg). Subjects may receive the pharmaceutical composition including sepiapterin, or a pharmaceutically acceptable salt thereof, once daily, twice daily or three times daily during treatment. In some embodiments, subjects may not be permitted to take any drugs known to inhibit folate synthesis (e.g., methotrexate, pemetrexed, or trimetrexate). Sepiapterin, or a pharmaceutically acceptable salt thereof, may be administered in two equal doses (e.g., two doses at different times of day), e.g., two 60 mg/kg doses (e.g., one 60 mg/kg dose in the morning and one 60 mg/kg dose in the evening), two 40 mg/kg doses (e.g., one 40 mg/kg dose in the morning and one 40 mg/kg dose in the evening), two 30 mg/kg doses (e.g., one 30 mg/kg dose in the morning and one 30 mg/kg dose in the evening), two 20 mg/kg doses (e.g., one 20 mg/kg dose in the morning and one 20 mg/kg dose in the evening), or two 10 mg/kg doses (e.g., one 10 mg/kg dose in the morning and one 10 mg/kg dose in the evening).

### Equivalents and Scope

Those skilled in the art will recognize or be able to ascertain using no more than routine experimentation, many equivalents to the specific embodiments in accordance with the invention described herein. The scope of the present invention is not intended to be limited to the above Description, but rather is as set forth in the appended claims.

### EXAMPLES

### Example 1.

Orthotopic glioblastoma tumors were established in nude (U87) and syngeneic (GL261) mouse models on Day 0 (N=13/group). Starting on Day 6 in the U87 mouse model, sepiapterin (SP) was orally administered for either 6 days (10 mg/kg sepiapterin) or daily (1 mg/kg sepiapterin) until end of experiment (60 days). Starting on Day 6 in the GL261 mouse model, SP was orally administered daily (10mg/kg) until the end of experiment (60 days). Radiation (IR) (5 fxs of 2 Gy) with 3D treatment planning and delivery was started on Day 13, ending on Day 17.

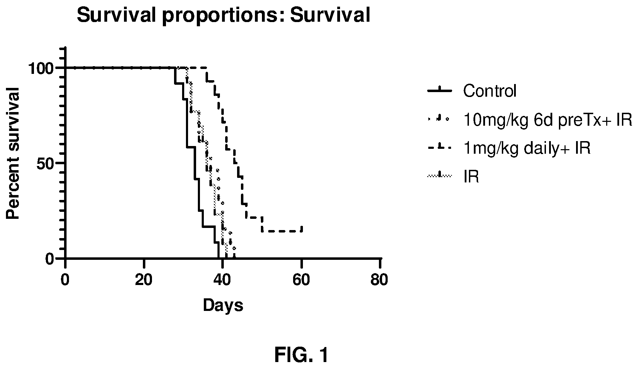

Results from U87 mice are shown in Figure 1. As shown in Figure 1, administration of sepiapterin improved survival over control (i.e. no IR or SP) or IR alone as follows: Both 10mg/kg for 6d+IR and 1mg/kg continuously+IR groups were significantly different from control group. The difference between the 10mg/kg for 6d group and the IR alone group did not achieve statistical significance. The 1mg/kg continuously group was significantly different from all other groups (Control, IR alone, and 10mg/kg for 6d).

Comparison of survival curves:
Log-rank (Mantel-Co) test: Chi square 34.39; df 3; P value <0.0001; P value summary ****; significant difference in survival curves.
Log-rank test for trend: Chi square 6.590; df 1; P value 0.0103; P value summary *; significant trend.
Gehan-Breslow-Wilcoxon test: Chi square 28.79; df 3; P value <0.0001; P value summary ****; significant difference in survival curves.

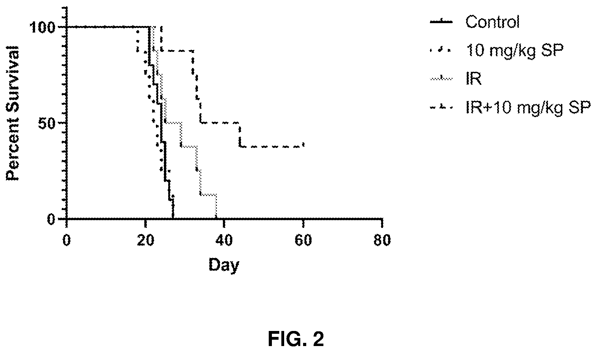

Results from the GL261 mouse model are shown in Figure 2. As shown in Figure 2, results from continuous administration of 10 mg/kg of SP were significantly different from control and IR alone.

Comparison of survival curves:
Log-rank (Mantel-Co) test: Chi square 21.32; df 3; P value <0.0001; P value summary ****; significant difference in survival curves.
Log-rank test for trend: Chi square 17.42; df 1; P value <0.0001; P value summary *; significant trend.
Gehan-Breslow-Wilcoxon test: Chi square 16.37; df 3; P value <0.0010; P value summary ****; significant difference in survival curves.

### Example 2:

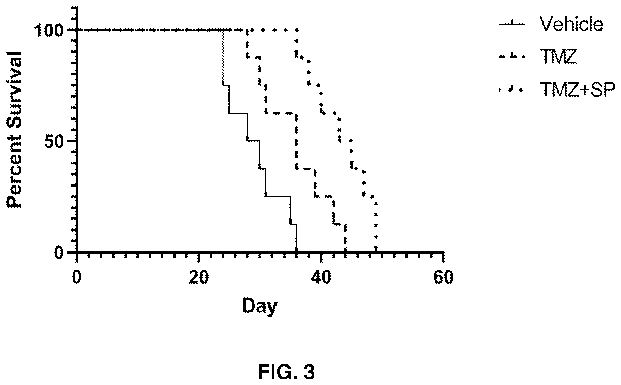

Orthotopic GL261 tumors were established on Day 0. Starting on Day 7 mice were treated with 60mg/kg temozolomide (TMZ) for 5 days +/- 10mg/kg SP given orally from day 7 until the end of the study. The results are shown in Figure 3. Figure 3 shows that mice receiving TMZ+SP had improved survival over mice receiving TMZ alone. Future animal studies will combine with fractionated radiotherapy.

### Example 3.

The novel object recognition test is a behavioral test in which a mouse is allowed to explore two similar objects during the first (training) session, and then after a certain length of time, one of the two training objects is replaced by a new object during a later (test) session. Because mice have an innate preference for novelty, if the mouse recognizes the familiar object, it will spend most of its time at the novel object. The amount of time taken to explore the new object provides a measure of recognition memory.

24 adult female C57BL/6J mice were treated as follows: (1) 8 mice were irradiated (IR) with 10 Gy total dose, divided into three fractions with a "rest" day in between fractions, covering about a 1mm square over the center of the head; (2) 8 mice were similarly irradiated, and also treated with 10 mg/kg/day sepiapterin (SP), administered as oral gavage, starting on day 1 of IR to 6 days beyond last radiation fraction (i.e. 11 days total); (3) 8 mice were untreated naïve.

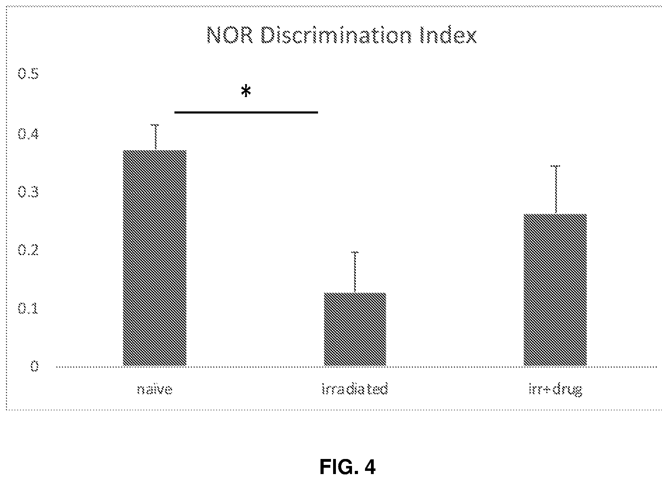

1 month following treatment, mice were tested in the novel object recognition (NOR) task with an hour inter-trial interval (ITI) to assess hippocampal mediated memory (i.e. the test occurred one hour after training). In both training and test phases, time investigating objects was measured for 5 minutes. Two independent observers were used to evaluate. The Discrimination Index = (novel - familiar)/(novel + familiar). As shown in Figure 4, administration of SP increased Discrimination Index compared with irradiated-only group.

One Way Analysis of Variance:
Normality Test (Shapiro-Wilk): Passed (P=0.992)
Equal Variance Test (Brown-Forsythe): Passed (P=0.540).

| **Group Name** | **N** | **Missing** | **Mean** | **Std Dev** | **SEM** |
|---|---|---|---|---|---|
| Naïve | 8 | 0 | 0.371 | 0.120 | 0.0423 |
| IR | 8 | 0 | 0.127 | 0.193 | 0.0682 |
| IR + drug | 8 | 0 | 0.262 | 0.227 | 0.0801 |

| **Source of Variation** | **DF** | **SS** | **MS** | **F** | **P** |
|---|---|---|---|---|---|
| Between groups | 2 | 0.240 | 0.120 | 3.500 | 0.049 |
| Residual | 21 | 0.720 | 0.0343 | | |
| Total | 23 | 0.960 | | | |

All Pairwise Multiple Comparison Procedures (Tukey Test):
Comparisons for factor: treatment group

| **Comparison** | **Diff of Means** | **p** | **q** | **P** | **P<0.050** |
|---|---|---|---|---|---|
| Naive vs. IR | 0.244 | 3 | 3.735 | 0.039 | Yes |
| Naive vs. IR + drug | 0.110 | 3 | 1.674 | 0.475 | No |
| IR + drug vs. IR | 0.135 | 3 | 2.061 | 0.331 | No |

2 months following treatment, mice are tested for spontaneous alternations in the y-maze. At 3 months following treatment, mice are tested again for NOR with a 1hr ITI.

### Example 4: RNA Study

The progressive, late delayed damage to the brain after high-dose radiation is thought to be caused by radiation-induced long-lived free radicals, reactive oxygen species, and pro-inflammatory cytokines. ICAM-1, TNF-α, IL-1β, and IFN-_{γ} are all upregulated in mouse brain following whole brain irradiation and have been implicated in the development of late injury in the brain. RNA extracts from brain tissue following total brain irradiation (TBI) of 10 Gy (3 fxs x 3.3 Gy) with and without 10mg/kg SP for 6 days are taken on days 8, 30 and 60 and analyzed for the above cytokines. As we have previously demonstrated in lung tissue, we expect the expression of these pro-inflammatory cytokines to increase after TBI exposure and that treatment with SP will normalize their expression.

### Example 5: Phase 2 study of Sepiapterin in Combination with Temozolomide in newly diagnosed or recurrent glioblastoma

### Summary

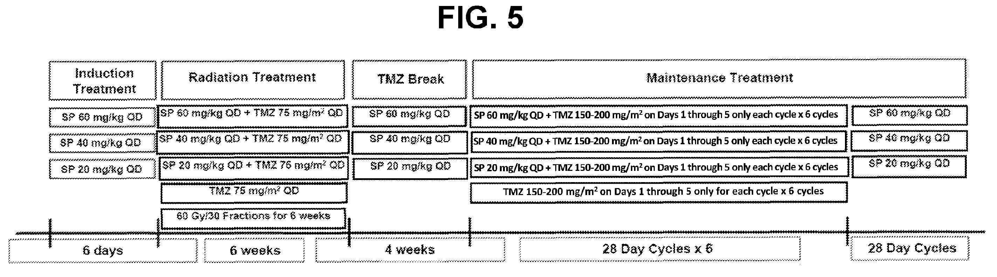

This is an open-label, randomized Phase 2 clinical trial to test the safety and effectiveness of sepiapterin (SP) for treating newly diagnosed or recurrent glioblastoma. The study design is shown in Figure 5. The purpose of this study is to test the safety profile and establish a recommended dose (RD) for phase 3 of sepiapterin plus standard temozolomide (TMZ) chemoradiotherapy in patients with newly diagnosed or recurrent glioblastoma. The study will test 3 dose levels of sepiapterin (20, 40, or 60 mg/kg/day) in combination with standard TMZ chemoradiotherapy versus standard TMZ chemoradiotherapy alone. Patients randomized to sepiapterin are treated for 6 days ("Induction Treatment" in Fig. 5) at 20 mg/kg/day, 40 mg/kg/day, or 60 mg/kg/day, as part of an induction treatment. This is followed by standard treatment consisting of radiotherapy of 60 Gy/30 fractions for 6 weeks plus 75 mg/m² TMZ daily, plus sepiapterin for the noted sepiapterin groups ("Radiation Treatment" in Fig. 5). This is followed by 4 weeks of treatment break for TMZ only with continuous treatment of sepiapterin ("TMZ break" in Figure 5), followed by maintenance treatment ("Maintenance Treatment" in Fig. 5) with 6 maintenance cycles of TMZ 150-200 mg/m² on Days 1 to 5 q28) combined with sepiapterin administered daily for each 28-day chemotherapy maintenance cycle. Patients randomized to TMZ only will be treated with standard treatment (consisting of radiotherapy of 60 Gy/30 fractions for 6 weeks plus 75 mg/m² TMZ daily, followed by 4 weeks of treatment break for TMZ, followed by maintenance treatment with 6 maintenance cycles of TMZ 150-200 mg/m² on Days 1 to 5 q28) of each 28-day chemotherapy maintenance cycle. See Figure 5 for study Schema.

### Objectives

### Primary Outcome Measure

- 6-month progression free survival (PFS) rate in all participants by RECIST Version 1.1

### Secondary Outcome Measures

- Overall Response Rate (ORR) in all patients by RECIST Version 1.1; OOR to be assessed by performing study imaging every 6-9 weeks after the first dose of study treatment. ORR is defined as the proportion of participants in the analysis population who had a complete response (CR) defined as a disappearance of all target lesions with pathological lymph nodes having a reduction in short axis to <10 mm) or partial response (PR) defined as at least a 30% decrease in the sum of the diameters of target lesions, using the baseline sum diameters as a reference.
- Disease control rate in all patients by RECIST Version 1.1; Disease control rate is defined as the proportions of patients whose best curative effect reaches complete remission, partial remission or disease control maintained for at least 4 weeks by RECIST Version 1.1.
- Overall Survival (OS) in all participants; OS defined as the time from the first day of study treatment to death due to any cause and analyzed by the Kaplan-Meier method for censored data and reported in months.
- Median PFS in all participants; median PFS defined as the median time from the first day of study treatment to the first documented Progressive Disease (PD) per RECIST Version 1.1 or death due to any cause, whichever occurs first.
- Median OS in all participants defined as the time from the first day of study treatment to death due to any cause.
- Safety as measured by Adverse Events (AE) inclusive of serious adverse events (SAE).

### Criteria

### Inclusion Criteria:

1. Voluntary participation and written informed consent
2. Subjects ≥18 years old
3. Supratentorial space occupying lesions diagnosed as glioblastoma by pathology;
4. Patients scheduled for standard radiotherapy and temozolomide concurrent chemotherapy after surgery
5. MRI confirmed newly diagnosis or that the tumor had definite recurrence. For recurrence, the diameter of the enhancement focus was more than 1 cm and more than 2 layers (layer spacing was 5 mm), or the recurrence was confirmed by pathology after re biopsy or operation;
6. According to RECIST Version 1.1, there was at least one measurable lesion;
7. Karnofsky Performance Status Scale (KPS) score ≥ 60 points;
8. Can swallow SEPIAPTERIN or TMZ normally;
9. The expected survival time is more than 3 months;
10. Sufficient organs and bone marrow function. The definition is as follows.
   a. Neutrophil count (ANC) ≥ 1500 / mm3 (1.5 ×109 / L);
   b. Platelet count (PLT) ≥ 100000 / mm3 (100 × 109 / L);
   c. Hemoglobin (HB) ≥ 9 g / dl (90 g / L);
   d. Serum albumin ≥ 2.8 g / dl;
   e. Serum creatinine ≤ 1.5 times the upper limit of normal value (ULN) or creatinine clearance rate ≥ 60 ml / min;
   f. Total bilirubin (TB) ≤ 1.5 × ULN, or total bilirubin (TB) > 1.5 × ULN, but direct bilirubin (DBIL) ≤ 1 × ULN; patients with liver metastasis should be ≤ 2 × ULN;
   g. The level of Aspartate Aminotransferase (AST) / Serum Glutamic-Oxaloacetic Transaminase (SGOT) or Alanine Transaminase (ALT) / Serum glutamic pyruvic transaminase (SGPT) should be ≤ 2.5 × ULN and ≤ 5 × ULN in patients with liver metastasis;
   h. Left ventricular ejection fraction (LVEF) ≥ 50%, Corrected Q-T interval (QTc) < 450 ms in male and < 470ms in female;
11. The international normalized ratio (INR) of prothrombin time is ≤1.5 and activated partial thromboplastin time (APTT) are ≤1.5 times the upper limit of normal value in patients who have not received anticoagulant therapy. Patients receiving full dose or parenteral anticoagulant therapy can enter the clinical trial as long as the dosage of anticoagulant drugs is stable for at least 2 weeks before entering the clinical study, and the results of coagulation test are within the limits of local treatment;
12. Women of childbearing age should have negative pregnancy test (serum or urine) within 7 days before enrollment, and voluntarily use appropriate contraceptive methods during the observation period and within 8 weeks after the last administration of the study drug; for men, it should be surgical sterilization or agree to use appropriate contraceptive methods during the observation period and within 8 weeks after the last administration of the study drug;
13. Good compliance, can cooperate with the study and follow-up according to the requirements of the program.

### Exclusion Criteria:

1. Previous allergic history of temozolomide or sepiapterin;
2. Major surgery (biopsy is allowed due to diagnosis; tumor resection is permitted if possible) or severe trauma within 4 weeks before the first use of the study drug;
3. Currently participating in other clinical studies, unless it is an observational (non intervention) clinical study or an intervention in the follow-up of a new clinical study; or has participated in any other drug clinical study within 4 weeks before the first administration, or no more than 5 half-life from the last study medication;
4. Except basal cell carcinoma or squamous cell carcinoma of skin, superficial bladder cancer, carcinoma in situ of cervix, intraductal carcinoma in situ of breast and papillary thyroid carcinoma which can be treated locally and have been cured in the past 5 years or at the same time;
5. Advanced patients with symptoms, spread to the viscera, and at risk of life-threatening complications in a short period of time (including patients with uncontrollable large amount of exudate [chest, pericardium, abdominal cavity];
6. Fever of unknown origin > 38.5°C occurred during the screening period / before the first administration (according to the researcher's judgment, fever caused by tumor can be included in the group);
7. Severe infection (CTCAE > Level 2) occurred within 4 weeks before the first use of the study drug, such as severe pneumonia, bacteremia, infection complications, etc. the baseline chest imaging examination revealed active pulmonary inflammation, symptoms and signs of infection within 2 weeks before the first use of the study drug, or the need for oral or intravenous antibiotic treatment (excluding prophylactic use of antibiotics)
8. Within 6 months before entering the study, the following conditions occurred: myocardial infarction, severe / unstable angina pectoris, New York Heart Association (NYHA) grade 2 or above cardiac insufficiency and clinically significant supraventricular or ventricular arrhythmias requiring clinical intervention; hypertension with poor drug control (systolic blood pressure continuously increased ≥ 150mmhg or diastolic blood pressure ≥ 100mmhg);
9. History of gastrointestinal bleeding or tendency of gastrointestinal bleeding in the past 6 months, such as esophageal varices, local active ulcer lesions, fecal occult blood≥(+) (gastroscopy is required when fecal occult blood is (+));
10. Unable to swallow the study drug, chronic diarrhea (including but not limited to irritable bowel syndrome, Crohn's disease, ulcerative colitis), intestinal obstruction and other factors affecting drug administration and absorption;
11. History of allogeneic organ transplantation or allogeneic hematopoietic stem cell transplantation or congenital immunodeficiency was known;
12. Patients with active pulmonary tuberculosis infection found through medical history or CT examination, or patients with active pulmonary tuberculosis infection within one year before enrollment, or patients with active pulmonary tuberculosis infection history one year ago but without regular treatment;
13. Human immunodeficiency virus (HIV) infection or acquired immunodeficiency syndrome (AIDS); untreated active hepatitis B (hepatitis B, defined as hepatitis B virus surface antigen [HBsAg] positive test results, HBV-DNA ≥ 500 Hepatitis C was defined as hepatitis C antibody [HCV AB] positive, HCV-RNA higher than the detection limit of analysis method and abnormal liver function), or combined with hepatitis B and hepatitis C co infection;
14. Patients had a clear history of neurological or mental disorders, including epilepsy and dementia, and was known to have a history of psychotropic substance abuse, alcoholism or drug abuse;
15. Patients who were considered unsuitable for the study.
16. Concomitant use of methotrexate or other antifolates.

## Claims

1. Sepiapterin, or a pharmaceutically acceptable salt thereof, for use in a method of treating glioblastoma in a subject,
the method comprising administering to the subject an effective amount of the sepiapterin or pharmaceutically acceptable salt thereof,
wherein the method further comprises:
treating the subject with therapeutic radiation; and/or
treating the subject with temozolomide (TMZ).

2. Sepiapterin, or a pharmaceutically acceptable salt thereof, for use according to claim 1, wherein:
the effective amount of sepiapterin, or pharmaceutically acceptable salt thereof, is about 10 mg/kg to about 60 mg/kg per dose;
optionally wherein the effective amount of sepiapterin, or pharmaceutically acceptable salt thereof, is:
about 20 mg/kg to about 60 mg/kg per dose;
about 20 mg/kg per dose;
about 40 mg/kg per dose; or
about 60 mg/kg per dose.

3. Sepiapterin, or a pharmaceutically acceptable salt thereof, for use according to claim 1 or 2, wherein:
the effective amount of sepiapterin, or pharmaceutically acceptable salt thereof, is administered once daily.

4. Sepiapterin, or a pharmaceutically acceptable salt thereof, for use according to any one of claims 1-3, wherein:
the effective amount of sepiapterin, or pharmaceutically acceptable salt thereof, is administered twice daily;
optionally wherein the effective amount of sepiapterin, or pharmaceutically acceptable salt thereof, is administered in two equal doses.

5. Sepiapterin, or a pharmaceutically acceptable salt thereof, for use according to any one of claims 1-4, wherein:
the effective amount of sepiapterin, or a pharmaceutically acceptable salt thereof, is administered with food;
optionally wherein:
the administration to the subject occurs less than 30 minutes prior to consuming food or after consuming food; or
the administration to the subject is substantially at the same time as food.

6. Sepiapterin, or a pharmaceutically acceptable salt thereof, for use according to claim 5, wherein:
the food is high protein and/or high fat food;
the food is a low-fat food; or
the food is high calorie food.

7. Sepiapterin, or a pharmaceutically acceptable salt thereof, for use according to any one of claims 1-6, wherein:
the effective amount of sepiapterin, or a pharmaceutically acceptable salt thereof, is administered without food;
optionally wherein:
the administration to the subject occurs more than 30 minutes prior to consuming food or more than 2 hours after consuming food; or
the administration to the subject occurs more than 30 minutes prior to consuming food or more than 3 hours after consuming food.

8. Sepiapterin, or a pharmaceutically acceptable salt thereof, for use according to any one of claims 1-7, wherein:
the sepiapterin or a pharmaceutically acceptable salt thereof, is formulated as an oral powder for suspension;
the sepiapterin or a pharmaceutically acceptable salt thereof, is administered as a suspension in a flavored suspending vehicle; or
the sepiapterin or a pharmaceutically acceptable salt thereof, is administered as a powder suspended in water or juice.

9. Sepiapterin, or a pharmaceutically acceptable salt thereof, for use according to any one of claims 1-8, wherein:
the method comprises treating the subject with therapeutic radiation.

10. Sepiapterin, or a pharmaceutically acceptable salt thereof, for use according to claim 9, wherein:
the sepiapterin or a pharmaceutically acceptable salt thereof is administered:
during the duration of the therapeutic radiation;
prior to the therapeutic radiation; or
subsequent to the therapeutic radiation.

11. Sepiapterin, or a pharmaceutically acceptable salt thereof, for use according to any one of claims 1-10, wherein:
the sepiapterin or a pharmaceutically acceptable salt thereof is administered for at least 6 days;
optionally wherein:
the sepiapterin or a pharmaceutically acceptable salt thereof is administered for at least 14 days; or
the sepiapterin or a pharmaceutically acceptable salt thereof is administered for at least 30 days.

12. Sepiapterin, or a pharmaceutically acceptable salt thereof, for use according to any one of claims 1-11, wherein:
the sepiapterin or a pharmaceutically acceptable salt thereof is administered for at least 14 days subsequent to the therapeutic radiation;
optionally wherein the sepiapterin or a pharmaceutically acceptable salt thereof is administered for at least 30 days subsequent to the therapeutic radiation.

13. Sepiapterin, or a pharmaceutically acceptable salt thereof, for use according to any one of claims 1-12, wherein:
the sepiapterin or a pharmaceutically acceptable salt thereof is administered continuously concurrent to the therapeutic radiation.

14. Sepiapterin, or a pharmaceutically acceptable salt thereof, for use according to any one of claims 1-13, wherein:
the sepiapterin or a pharmaceutically acceptable salt thereof is administered continuously in 28-day cycles;
optionally wherein:
the sepiapterin or a pharmaceutically acceptable salt thereof is administered:
for six 28-day cycles; or
for more than six 28-day cycles.

15. Sepiapterin, or a pharmaceutically acceptable salt thereof, for use according to any one of claims 1-14, wherein:
the subject is a human.

## Patentansprüche

1. Sepiapterin oder ein pharmazeutisch annehmbares Salz davon zur Verwendung in einem Verfahren zur Behandlung eines Glioblastoms bei einem Individuum,
wobei das Verfahren das Verabreichen einer wirksamen Menge des Sepiapterins oder des pharmazeutisch annehmbaren Salzes davon an das Individuum umfasst;
wobei das Verfahren weiters Folgendes umfasst:
das Behandeln des Individuums mit therapeutischer Bestrahlung und/oder
das Behandeln des Individuums mit Temozolmid (TMZ).

2. Sepiapterin oder ein pharmazeutisch annehmbares Salz davon zur Verwendung nach Anspruch 1, wobei:
die wirksame Menge an Sepiapterin oder dem pharmazeutisch annehmbaren Salz davon etwa 10 mg/kg bis etwa 60 mg/kg pro Dosis beträgt;
wobei die wirksame Menge an Sepiapterin oder dem pharmazeutisch annehmbaren Salz davon gegebenenfalls die folgende ist:
etwa 20 mg/kg bis etwa 60 mg/kg pro Dosis,
etwa 20 mg/kg pro Dosis,
etwa 40 mg/kg pro Dosis oder
etwa 60 mg/kg pro Dosis.

3. Sepiapterin oder ein pharmazeutisch annehmbares Salz davon zur Verwendung nach Anspruch 1 oder 2, wobei:
die wirksame Menge an Sepiapterin oder dem pharmazeutisch annehmbaren Salz davon einmal täglich verabreicht wird.

4. Sepiapterin oder ein pharmazeutisch annehmbares Salz davon zur Verwendung nach einem der Ansprüche 1 bis 3, wobei:
die wirksame Menge an Sepiapterin oder dem pharmazeutisch annehmbaren Salz davon zweimal täglich verabreicht wird;
wobei die wirksame Menge von Sepiapterin oder dem pharmazeutisch annehmbaren Salz davon gegebenenfalls in zwei gleichen Dosen verabreicht wird.

5. Sepiapterin oder ein pharmazeutisch annehmbares Salz davon zur Verwendung nach einem der Ansprüche 1 bis 4, wobei:
die wirksame Menge an Sepiapterin oder einem pharmazeutisch annehmbaren Salz davon zusammen mit einer Mahlzeit verabreicht wird;
wobei gegebenenfalls:
die Verabreichung an das Individuum weniger als 30 min vor dem Verzehr der Mahlzeit oder nach dem Verzehr der Mahlzeit erfolgt oder
die Verabreichung an das Individuum im Wesentlichen gleichzeitig mit der Mahlzeit erfolgt.

6. Sepiapterin oder ein pharmazeutisch annehmbares Salz davon zur Verwendung nach Anspruch 5, wobei:
die Mahlzeit proteinreich und/oder fettreich ist,
die Mahlzeit fettarm ist oder
die Mahlzeit hochkalorisch ist.

7. Sepiapterin oder ein pharmazeutisch annehmbares Salz davon zur Verwendung nach einem der Ansprüche 1 bis 6, wobei:
die wirksame Menge an Sepiapterin oder einem pharmazeutisch annehmbaren Salz davon ohne eine Mahlzeit verabreicht wird;
wobei gegebenenfalls:
die Verabreichung an das Individuum mehr als 30 min vor dem Verzehr der Mahlzeit oder mehr als 2 h nach Verzehr der Mahlzeit erfolgt oder
die Verabreichung an das Individuum mehr als 30 min vor dem Verzehr der Mahlzeit der mehr als 3 h nach dem Verzehr der Mahlzeit erfolgt.

8. Sepiapterin oder ein pharmazeutisch annehmbares Salz davon zur Verwendung nach einem der Ansprüche 1 bis 7, wobei:
das Sepiapterin oder ein pharmazeutisch annehmbares Salz davon zu einem oralen Pulver zur Suspension formuliert ist,
das Sepiapterin oder ein pharmazeutisch annehmbares Salz davon als Suspension in einem mit Geschmack versehenen Suspendiervehikel verabreicht wird oder
das Sepiapterin oder ein pharmazeutisch annehmbares Salz davon als in Wasser oder Saft suspendiertes Pulver verabreicht wird.

9. Sepiapterin oder ein pharmazeutisch annehmbares Salz davon zur Verwendung nach einem der Ansprüche 1 bis 8, wobei:
das Verfahren das Behandeln des Individuums mit therapeutischer Bestrahlung umfasst.

10. Sepiapterin oder ein pharmazeutisch annehmbares Salz davon zur Verwendung nach Anspruch 9, wobei:
das Sepiapterin oder ein pharmazeutisch annehmbares Salz davon wie folgt verabreicht wird:
während der Dauer der therapeutischen Bestrahlung,
vor der therapeutischen Bestrahlung oder
nach der therapeutischen Bestrahlung.

11. Sepiapterin oder ein pharmazeutisch annehmbares Salz davon zur Verwendung nach einem der Ansprüche 1 bis 10, wobei:
das Sepiapterin oder ein pharmazeutisch annehmbares Salz davon zumindest 6 Tage lang verabreicht wird;
wobei gegebenenfalls:
das Sepiapterin oder ein pharmazeutisch annehmbares Salz davon zumindest 14 Tage lang verabreicht wird oder
das Sepiapterin oder ein pharmazeutisch annehmbares Salz davon zumindest 30 Tage lang verabreicht wird.

12. Sepiapterin oder ein pharmazeutisch annehmbares Salz davon zur Verwendung nach einem der Ansprüche 1 bis 11, wobei:
das Sepiapterin oder ein pharmazeutisch annehmbares Salz davon nach der therapeutischen Bestrahlung zumindest 14 Tage lang verabreicht wird;
wobei das Sepiapterin oder ein pharmazeutisch annehmbares Salz davon nach der therapeutischen Bestrahlung gegebenenfalls zumindest 30 Tage lang verabreicht wird.

13. Sepiapterin oder ein pharmazeutisch annehmbares Salz davon zur Verwendung nach einem der Ansprüche 1 bis 12, wobei:
das Sepiapterin oder ein pharmazeutisch annehmbares Salz davon kontinuierlich gleichzeitig mit der therapeutischen Bestrahlung verabreicht wird.

14. Sepiapterin oder ein pharmazeutisch annehmbares Salz davon zur Verwendung nach einem der Ansprüche 1 bis 13, wobei:
das Sepiapterin oder ein pharmazeutisch annehmbares Salz davon kontinuierlich in Zyklen von 28 Tagen verabreicht wird;
wobei gegebenenfalls:
das Sepiapterin oder ein pharmazeutisch annehmbares Salz davon 6 Zyklen von 28 Tagen lang verabreicht wird oder
mehr als 6 Zyklen von 28 Tagen lang verabreicht wird.

15. Sepiapterin oder ein pharmazeutisch annehmbares Salz davon zur Verwendung nach einem der Ansprüche 1 bis 14, wobei:
das Individuum ein Mensch ist.

## Revendications

1. Sépiaptérine, ou un sel pharmaceutiquement acceptable de celle-ci, pour utilisation dans un procédé de traitement du glioblastome chez un sujet,
le procédé comprenant une administration au sujet d'une quantité efficace de la sépiaptérine ou d'un sel pharmaceutiquement acceptable de celle-ci,
dans lequel le procédé comprend en outre les étapes consistant à :
traiter le sujet avec un rayonnement thérapeutique ;
et/ou
traiter le sujet avec du témozolomide (TMZ).

2. Sépiaptérine, ou un sel pharmaceutiquement acceptable de celle-ci, pour utilisation selon la revendication 1, dans laquelle :
la quantité efficace de sépiaptérine, ou d'un sel pharmaceutiquement acceptable de celle-ci, est d'environ 10 mg/kg à environ 60 mg/kg par dose ;
facultativement dans laquelle la quantité efficace de sépiaptérine, ou d'un sel pharmaceutiquement acceptable de celle-ci, est :
d'environ 20 mg/kg à environ 60 mg/kg par dose ;
d'environ 20 mg/kg par dose ;
d'environ 40 mg/kg par dose ;
soit environ 60 mg/kg par dose.

3. Sépiaptérine, ou un sel pharmaceutiquement acceptable de celle-ci, pour utilisation selon la revendication 1 ou 2, dans laquelle :
la quantité efficace de sépiaptérine, ou d'un sel pharmaceutiquement acceptable de celle-ci, est administrée une fois par jour.

4. Sépiaptérine, ou un sel pharmaceutiquement acceptable de celle-ci, pour utilisation selon l'une quelconque des revendications 1 à 3, dans laquelle :
la quantité efficace de sépiaptérine, ou d'un sel pharmaceutiquement acceptable de celle-ci, est administrée deux fois par jour ;
facultativement dans laquelle la quantité efficace de sépiaptérine, ou d'un sel pharmaceutiquement acceptable de celle-ci, est administrée en deux doses égales.

5. Sépiaptérine, ou un sel pharmaceutiquement acceptable de celle-ci, pour utilisation selon l'une quelconque des revendications 1 à 4, dans laquelle :
la quantité efficace de sépiaptérine, ou d'un sel pharmaceutiquement acceptable de celle-ci, est administrée avec des aliments ;
facultativement dans laquelle :
l'administration au sujet a lieu moins de 30 minutes avant de consommer des aliments ou après avoir consommé des aliments ; ou
l'administration au sujet est sensiblement en même temps qu'un aliment.

6. Sépiaptérine, ou un sel pharmaceutiquement acceptable de celle-ci, pour utilisation selon la revendication 5, dans laquelle :
l'aliment est un aliment riche en protéines et/ou en matières grasses ;
l'aliment est un aliment faible en matières grasses ; ou
l'aliment est un aliment riche en calories.

7. Sépiaptérine, ou un sel pharmaceutiquement acceptable de celle-ci, pour utilisation selon l'une quelconque des revendications 1 à 6, dans laquelle :
la quantité efficace de sépiaptérine, ou d'un sel pharmaceutiquement acceptable de celle-ci, est administrée sans aliment ;
facultativement dans laquelle :
l'administration au sujet a lieu plus de 30 minutes avant de consommer des aliments ou plus de 2 heures après avoir consommé des aliments ; ou
l'administration au sujet a lieu plus de 30 minutes avant de consommer des aliments ou plus de 3 heures après avoir consommé des aliments.

8. Sépiaptérine, ou un sel pharmaceutiquement acceptable de celle-ci, pour utilisation selon l'une quelconque des revendications 1 à 7, dans laquelle :
la sépiaptérine, ou un sel pharmaceutiquement acceptable de celle-ci, est formulée sous forme de poudre orale pour suspension ;
la sépiaptérine, ou un sel pharmaceutiquement acceptable de celle-ci, est administrée sous forme de suspension dans un excipient de suspension aromatisé ; ou
la sépiaptérine, ou un sel pharmaceutiquement acceptable de celle-ci, est administrée sous forme de poudre en suspension dans de l'eau ou du jus.

9. Sépiaptérine, ou un sel pharmaceutiquement acceptable de celle-ci, pour utilisation selon l'une quelconque des revendications 1 à 8, dans laquelle :
le procédé comprend le traitement du sujet avec un rayonnement thérapeutique.

10. Sépiaptérine, ou un sel pharmaceutiquement acceptable de celle-ci, pour utilisation selon la revendication 9, dans laquelle :
la sépiaptérine, ou un sel pharmaceutiquement acceptable de celle-ci, est administrée :
pendant la durée du rayonnement thérapeutique ;
avant le rayonnement thérapeutique ; ou
après le rayonnement thérapeutique.

11. Sépiaptérine, ou un sel pharmaceutiquement acceptable de celle-ci, pour utilisation selon l'une quelconque des revendications 1 à 10, dans laquelle :
la sépiaptérine, ou un sel pharmaceutiquement acceptable de celle-ci, est administrée pendant au moins 6 jours ;
facultativement dans laquelle :
la sépiaptérine, ou un sel pharmaceutiquement acceptable de celle-ci, est administrée pendant au moins 14 jours ; ou
la sépiaptérine, ou un sel pharmaceutiquement acceptable de celle-ci, est administrée pendant au moins 30 jours.

12. Sépiaptérine, ou un sel pharmaceutiquement acceptable de celle-ci, pour utilisation selon l'une quelconque des revendications 1 à 11, dans laquelle :
la sépiaptérine, ou un sel pharmaceutiquement acceptable de celle-ci, est administrée pendant au moins 14 jours après le rayonnement thérapeutique ;
facultativement dans laquelle la sépiaptérine, ou un sel pharmaceutiquement acceptable de celle-ci, est administrée pendant au moins 30 jours après le rayonnement thérapeutique.

13. Sépiaptérine, ou un sel pharmaceutiquement acceptable de celle-ci, pour utilisation selon l'une quelconque des revendications 1 à 12, dans laquelle :
la sépiaptérine, ou un sel pharmaceutiquement acceptable de celle-ci, est administrée en continu en même temps que le rayonnement thérapeutique.

14. Sépiaptérine, ou un sel pharmaceutiquement acceptable de celle-ci, pour utilisation selon l'une quelconque des revendications 1 à 13, dans laquelle :
la sépiaptérine, ou un sel pharmaceutiquement acceptable de celle-ci, est administrée en continu par cycles de 28 jours ;
facultativement dans laquelle :
la sépiaptérine, ou un sel pharmaceutiquement acceptable de celle-ci, est administrée :
pendant six cycles de 28 jours ; ou
pendant plus de six cycles de 28 jours.

15. Sépiaptérine, ou un sel pharmaceutiquement acceptable de celle-ci, pour utilisation selon l'une quelconque des revendications 1 à 14, dans laquelle :
le sujet est un être humain.
